# EUROPEAN PATENT APPLICATION

(11) **EP 4 353 706 A1**
(43) Date of publication of application: **17.04.2024**
(21) Application number: 22806814.4
(22) Date of filing: 11.05.2022
(51) Int. Cl.: C07C 39/08, C07C 39/19, C07C 39/24, A61K 31/05, A61P 3/00

(54) **CLASS OF ALKYLPHENOL COMPOUNDS AND PREPARATION METHOD THEREFOR**

(30) Priority: 11.05.2021 CN 202110513466
(71) Applicant: Shanghai Institute of Materia Medica, Chinese Academy of Sciences, Pudong, Shanghai 201203 (CN)
(72) Inventor: NAN, Fajun, Shanghai 201203 (CN); LI, Jingya, Shanghai 201203 (CN); ZHANG, Zheng, Shanghai 201203 (CN); DUAN, Yanan, Shanghai 201203 (CN); ZHANG, Yangming, Shanghai 201203 (CN); CHEN, Dandan, Shanghai 201203 (CN); DAI, Chengqiu, Shanghai 201203 (CN)
(74) Representative: Lavoix
(86) International application number: PCT/CN2022/092317
(87) International publication number: WO 2022/237852

(57) **Abstract**

Provided in the present invention are a class of alkylphenol compounds and a preparation method therefor. Specifically provided in the present invention are a new alkyl polyphenol compound as represented by chemical formula I, and a preparation method therefor and the use thereof in the treatment of metabolic syndrome.

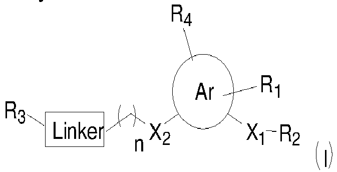

## Description

### Technical field

The present invention belongs to pharmaceutical field, specifically, the present invention relates to a class of structurally novel alkylphenol compounds, a preparation method therefor, and a use of the compound as DRAK2 kinase inhibitors in the treatment of metabolic syndrome drugs.

### Background

Death Associated Related Apoptotic Protein Kinase 2 (DRAK2) is a threonine/serine kinase and a member of the Death associated Protein Kinase (DAPK) family (J. Biol. Chem. 1998, 273, 29066). DAPK family members play a crucial role in various cell death signaling pathways. In addition to DRAK2, four other DAP kinase family members have been identified so far, including DAPK1, DAPK2, DAPK3, and DRAK1. The amino acid sequence homology of the five DAPK members is strictly limited to the N-terminal kinase region, while the C-terminal region is unique, and its uniqueness is required for each kinase's regulatory function (Med. Res. Rev. 2019, 39, 349).

Diabetes is a metabolic disease, mainly including type 1 diabetes and type 2 diabetes. Type 1 diabetes (T1D) is a chronic autoimmune disease that causes damage to pancreatic β cells, leading to insulin deficiency and thus hyperglycemia. The pathogenesis of type 2 (T2D) diabetes is more complex, mainly due to the inability of the patient's pancreatic β-cells to produce enough insulin to meet body needs, accompanied by insulin resistance and pancreatic β-cell dysfunction. Christopher confirmed that as the disease progresses, the persistent damage and attenuation of β cells of T2D patients are the fundamental factors in the deterioration of the disease. Therefore, pancreatic β cell apoptosis is present in both T1D and T2D. In addition, high sugar, high fat, and certain cytokines induce apoptosis in pancreatic β-cells through insulin receptor substrate-2 (IRS-2) signaling pathway(Science. 2005, 307, 380).

Studies have shown that high sugar, high fat (J. Cell. Biochem. 2008, 105, 1073), and certain cytokines (J. Immunol. 2009, 182, 4762) can promote high expression of DRAK2 at the cellular level, leading to an increased apoptosis of pancreatic β-cells. As verified by antisense RNA, the increase in β-cells apoptosis is closely related to DRAK2 gene, suggesting that DRAK2 may play a mediating role in the development of diabetes induced by external stimuli. Therefore, small molecule inhibitors targeting DRAK2 have the potential to treat diabetes. However, there are few reports on small molecule inhibitors of DRAK2 at this stage. The first DRAK2 inhibitor is SC82510, which was reported in 2014 (Neurochem. Res. 2014, 39, 403), but its chemical structure is unknown. This compound can inhibit DRAK2 at a low IC₅₀ value and induce axonal regeneration and branching at a concentration of 1 nM, but the specific mechanism is unknown.

There are currently three main types of DRAK2 inhibitors reported. The first type is 5-arylthieno[2,3-b] pyridine compounds, and its representative compound 22b has a moderate inhibitory rate on DRAK2 with an IC₅₀ value of 0.86 µM (J. Med. Chem. 2014, 57, 7624); the second type is indirubin-3'- monooxime compounds, which have a good inhibitory effect on the activity of DRAK2, and its representative compound 16 has an IC₅₀ of 3 nM (Bioorg. Med. Chem. Lett. 2016, 26, 2719); the third type is 2-benzylidene benzofuran-3-ketones, and its representative compound 41 has an IC50 of 0.25µ M (Eur. J. Med. Chem. 2017, 130195). However, no further in-depth research and development has been conducted on the abovementioned three types of compounds. In view of the potential of DRAK2 inhibitors in the treatment of metabolic syndrome such as diabetes, the development of novel DRAK2 small molecule inhibitors is highly valuable.

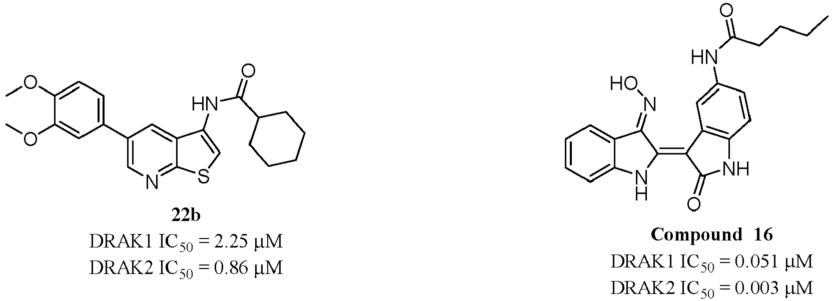

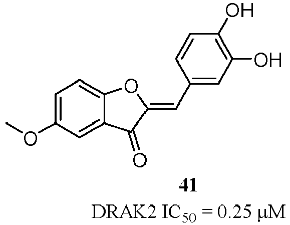

### SUMMARY OF THE INVENTION

The purpose of the present invention is to provide a novel alkyl polyphenol compound that can serve as a DRAK2 small molecule inhibitor with a novel structural framework. These compounds can be used to develop therapeutic drugs for metabolic syndrome, including but not limited to: diabetes, hyperlipidemia, obesity, nonalcoholic fatty liver, and biliary cirrhosis.

In the first aspect of the present invention, provided is a compound of formula I, or a pharmaceutically acceptable salt thereof, 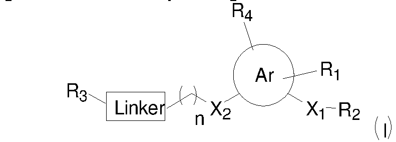 wherein,
R₁ is selected from the group consisting of hydrogen, hydroxyl, halogen, substituted or unsubstituted amino, and substituted or unsubstituted C1-C6 alkoxy;
R₂ is selected from the group consisting of hydrogen, CN, C1-C8 alkyl, 5-7 membered heteroaryl, 3-7 membered cycloalkane, C2-C10 alkenyl, -P(O)(ORa)₂, -SO₂Rb, and -(C=O)Rc, preferably hydrogen, cyano, methyl, 5-7 membered heteroaryl, - P(O)(ORa)₂, -SO₂Rb, and -(C=O)Rc, more preferably cyano, pyridine ring, substituted benzene ring, -P(O)(ORa)₂, - SO₂Rb, and -(C=O)Rc;
Rₐ is independently selected from the group consisting of C1-C8 alkyl, C3-C10 cycloalkyl, 5-7-membered heteroaryl, and C2-C10 alkenyl, preferably C1-C8 alkyl;
R_{b} is independently selected from the group consisting of C1-C8 alkyl, C3-C10 cycloalkyl, 5-7-membered heteroaryl, and C2-C10 alkenyl, preferably C1-C8 alkyl, C3-C10 cycloalkyl, and 5-7-membered heteroaryl, more preferably vinyl, C1-C3 alkyl, C3-C6 cycloalkyl, and 5-membered heteroaryl;
R_{c} is independently selected from the group consisting of C1-C8 alkyl, C3-C10 cycloalkyl, 5-7-membered heteroaryl, and C2-C10 alkenyl, preferably methyl and vinyl;
R₃ is selected from the group consisting of H, halogen, C1-C10 alkyl, and C3-C6 cycloalkyl;
R₄ is selected from the group consisting of H, OH, and substituted or unsubstituted C1-C8 alkoxy, preferably OH or methoxy;
Ar is selected from the group consisting of substituted or unsubstituted benzene ring,
   substituted or unsubstituted 5-9 membered heteroaryl, 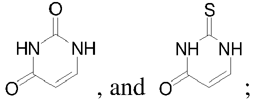
X₁ is a chemical bond, O, NH, NHC(O), NHS(O)₂, NHC(O)NH, NHS(O)₂NH or S;
X₂ is selected from the group consisting of chemical bond, C1-C6 alkylaminocarbonyl, and C1-C4 alkylcarbonyl;
n is 0-10, preferably 0-6;
Linker is selected from the group consisting of substituted or unsubstituted C1-C6 alkane, substituted or unsubstituted C1-C6 olefin, substituted or unsubstituted benzene ring, substituted or unsubstituted 5-7-membered heteroaromatic ring, and substituted or unsubstituted 3-6 membered cycloalkane, preferably C1-C6 olefin, substituted benzene ring, thiophene ring, and furan ring, more preferably olefin and benzene ring;
unless otherwise specified, the term "substituted" refers to the hydrogen atoms on the group is substituted by one or more substituents selected from the group consisting of halogen, hydroxyl, carboxyl, C1-C6 alkyl, and C2-C10 ester group.

In another preferred embodiment, Ar is selected from the group consisting of substituted or unsubstituted benzene ring, substituted or unsubstituted pyridine ring, substituted or unsubstituted indole ring, substituted or unsubstituted pyrimidine ring, substituted or unsubstituted benzimidazole, substituted or unsubstituted indazole, and substituted or unsubstituted benzotriazole, preferably benzene ring or indole ring.

In another preferred embodiment, R₁ is selected from the group consisting of hydrogen, fluorine, and chlorine.

In another preferred embodiment, R₂ is selected from the group consisting of hydrogen, cyano, methyl, 5-7-membered heteroaryl, -P(O)(ORa)₂, -SO₂Rb, and -(C=O)Rc;
Ra is independently selected from the group consisting of C1-C8 alkyl;
R_{b} is independently selected from the group consisting of vinyl, C1-C3 alkyl, C3-C6 cycloalkyl, and 5-membered heteroaryl;
Rc is independently selected from the group consisting of methyl and vinyl.

In another preferred embodiment, R₂ is selected from the group consisting of cyano, pyridine ring, substituted benzene ring, -P(O)(ORa)₂, -SO₂Rb, and -(C=O)R_{c};

In another preferred embodiment, R₃ is selected from the group consisting of hydrogen, halogen, isopropyl and cyclohexane.

In another preferred embodiment, X₂ is selected from the group consisting of a chemical bond, NHS(O)₂, and -C(=O)-NH-.

In another preferred embodiment, the compound of formula I has the structure shown in formula II as follows:

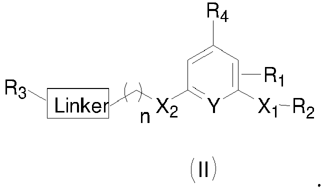

In another preferred example, the compound is selected from the group consisting of:

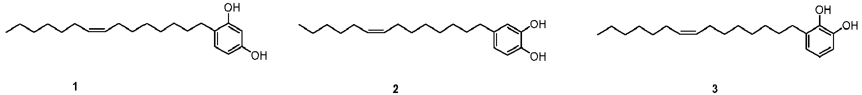

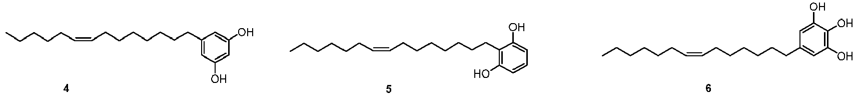

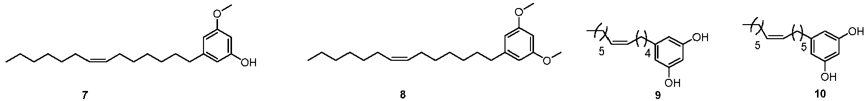

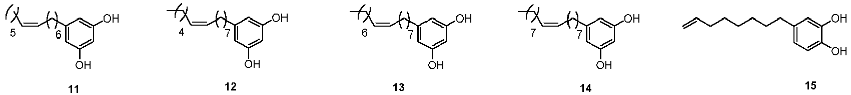

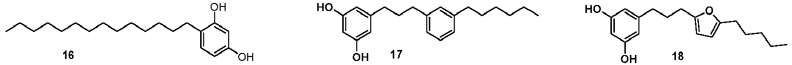

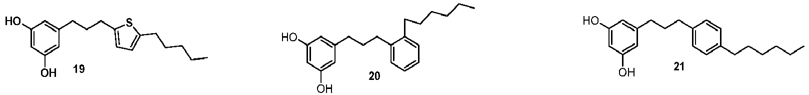

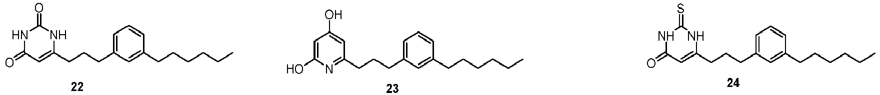

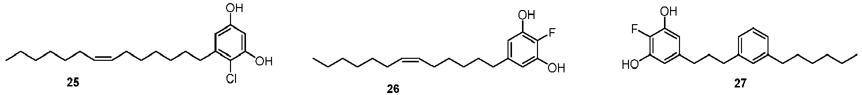

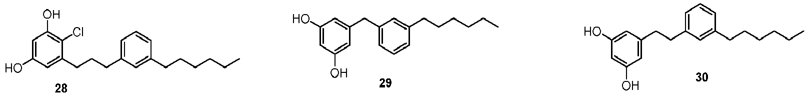

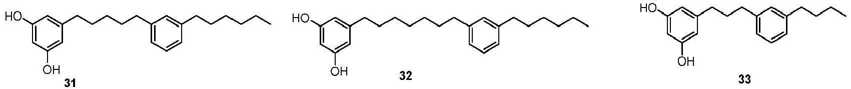

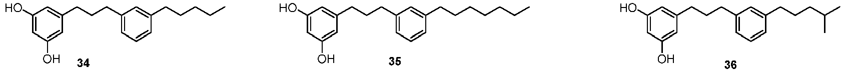

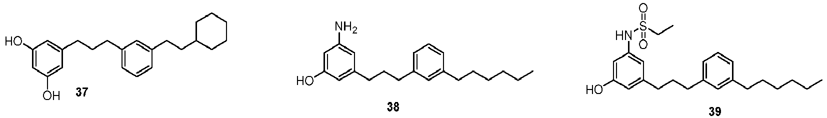

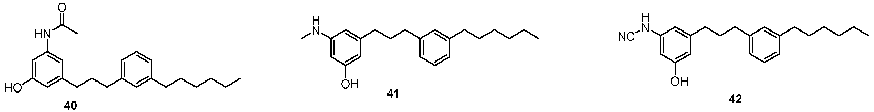

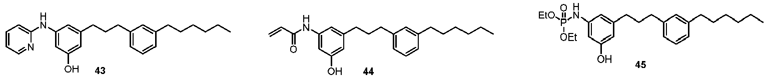

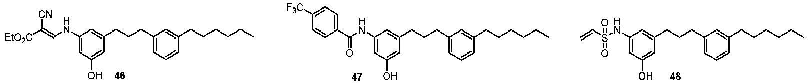

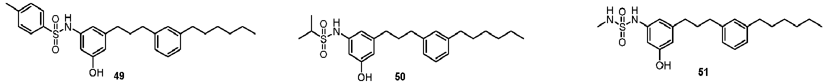

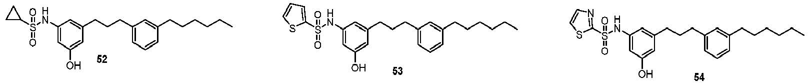

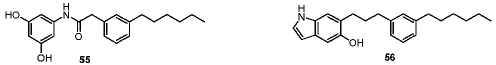

In a second aspect of the present invention, provided is a use of the compound according to the first aspect of the present invention in the preparation of a pharmaceutical composition for the treatment or prevention of indications associated with the activity or expression of death associated related apoptotic protein kinase 2 (DRAK2).

In another preferred example, the indication is metabolic syndromes.

In another preferred example, the metabolic syndrome includes but is not limited to: diabetes, hyperlipidemia, obesity, nonalcoholic fatty liver, and biliary cirrhosis.

In a third aspect of the present invention, provided is a pharmaceutical composition, comprising (1) the compound according to the first aspect, or pharmaceutically acceptable salts thereof as an active ingredient; and (2) pharmaceutically acceptable carriers.

It should be understood that, within the scope of the present invention, each of the above technical features of the present invention and each of the technical features specifically described in the following (such as the examples) can be combined with each other to constitute a new or preferred technical solution. Due to space limitations, It will not be repeated herein.

### EMBODIMENTS FOR CARRYING OUT THE INVENTION

After extensive and in depth research, the inventor of this application developed for the first time a class of compounds designed based on alkylphenol parent nucleus, which have good inhibitory effects on DRAK2 protein. Given the limited number of small molecule DRAK2 inhibitors reported so far, the novelty of alkyl polyphenol compound backbone holds great value for further in-depth research, and could provide a worthwhile direction for the ensuing study of novel small-molecule DRAK2 inhibitors. Based on the above discoveries, the inventors have completed the present invention.

### TERMS

As used herein, the alkyl described herein is preferably an aliphatic alkyl, which can be a straight chain alkyl or a branched chain alkyl, and non-limitingly includes: methyl, ethyl, n-propyl, isopropyl, n-butyl, isobutyl, tert butyl, etc. The expression "C1-C8" is intended to include the corresponding groups having 1, 2, 3, 4, 5, 6, 7 or 8 carbon atoms. For example, "C1-C8 alkyl" refers to an alkyl group with 1, 2, 3, 4, 5, 6, 7 or 8 carbon atoms, and "C2-C10 alkenyl" refers to an alkenyl group with 2, 3, 4, 5, 6, 7, 8, 9 or 10 carbon atoms.

As used herein, alkenyl is preferably vinyl, prropenyl, butenyl, styryl, phenylpropenyl, and the like.

The "aryl" refers to a 6 to 10 membered all carbon mono-cyclic or fused poly-cyclic (i.e. a ring that shares adjacent pairs of carbon atoms) group, and the group has a conjugated π electron system, such as phenyl and naphthalene. The aryl may be fused with a heterocyclyl, heteroaryl, or cycloalkyl ring, and non limiting examples include benzimidazole, benzothiazole, benzoxazole, benzisoxazole, benzopyrazole, quinoline, benzindole, and benzodihydrofuran.

The "heteroaryl" refers to a heteroaromatic system consisting of 1 to 4 heteroatoms and 5 to 14 ring atoms, wherein the heteroatom is selected from the group consisting of: oxygen, sulfur, and nitrogen. The heteroaryl is preferably 5 to 10 membered, such as furanyl, thienyl, pyridinyl, pyrrolyl, N-alkyl pyrrolyl, pyrimidinyl, pyrazinyl, imidazolyl, tetrazolyl, and the like. The heteroaryl can be fused with aryl, heterocyclyl or cycloalkyl ring, wherein the ring connected to the parent structure is heteroaryl.

As used herein, unless otherwise indicated, 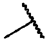 represents a connecting site.

Unless otherwise specified, the structural formula described in the present invention is intended to include all tautomeric, optical isomeric, and stereoisomeric forms (e. g., enantiomer, diastereomer, geometrical isomers or conformational isomers): for example, (Z), (E) isomers containing double bonds, and conformational isomers of (Z), (E). Thus the individual stereochemical isomers, tautomeric isomers of the compounds of the present invention or mixtures of enantiomers, diastereomers or geometrical isomers or conformational isomers or reciprocal isomers thereof fall within the scope of the present invention.

The term "tautomeric isomer" indicates that structural isomers with different energies can exceed low energy barriers and thus transform into each other. For example, proton tautomers (i.e. proton shift) include tautomerism through proton transfer, such as 1H-indazole and 2H-indazole, 1H-benzo [d] imidazole and 3H-benzo [d] imidazole, and valence tautomers include tautomerism through recombination of some bonding electrons.

### Preparation method

The compounds of the present invention can be prepared using the following route:
In the second aspect of the present invention, provided is a method for preparing a compound as described in the first aspect, or a pharmaceutically acceptable salt thereof. When the Linker is a cis double bond, the synthesis route of the target compound starts with alkyl-ynyl **H1** as the starting material with tetrahydrofuran as a solvent, followed by the slow addition of n-butyl lithium at -78°C, and then continues to add a solution of dibromoalkane**H2** in tetrahydrofuran to react overnight to obtain compound **H3.** Next, in the presence of nickel acetate and sodium borohydride, **H3** is reduced by hydrogenation with ethanol as solvent to give compound **H4.** In the presence of cuprous iodide, triphenylphosphine, and lithium methoxide, **H4** is coupled with bis(pinacolato)diboron at room temperature to obtain intermediate **H5. H5** and intermediate **H6** react overnight under the condition of NaO^{t}Bu, Ruphos, and Pd₂(dba)₃ in a mixed solvent of toluene and water at 80°C to obtain compound **H7.** Finally, the target compound **H8** can be obtained by removing the benzyl group of **H7** under the condition of N, N-dimethylaniline and aluminum trichloride. Wherein, n and m is 1-10; X is oxygen, sulfur, and nitrogen. In one embodiment, the compound or a pharmaceutically acceptable salt thereof is prepared by the following route:

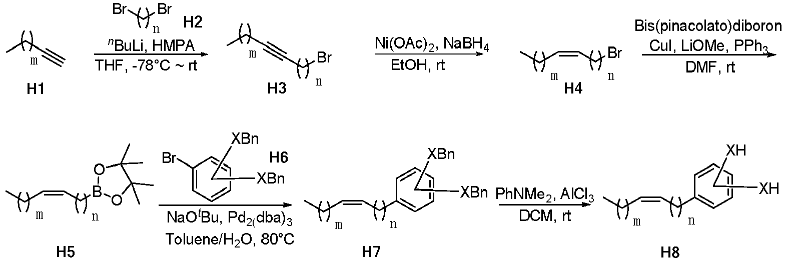

When the Linker is benzene ring, the synthesis route of the target compound is that halohydrin **H9** reacts with 3,4-dihydro-2H-pyran under the catalytic conditions of p-toluenesulfonic acid to obtain intermediate **H10.** In the presence of cuprous iodide, triphenylphosphine, and lithium methoxide, **H10** is coupled with bis(pinacolato)diboron at room temperature to obtain intermediate **H11**. **H11** undergoes Suzuki coupling reaction with substituted bromobenzene**H12** to obtain intermediate **H13,** which is then deprotected to obtain intermediate **H14. H14** undergoes Appel reaction to obtain brominated intermediate **H15,** which is then coupled with bis(pinacolato)diboron to form intermediate **H16. H16** undergoes Suzuki coupling reaction to obtain intermediate **H17.** Finally, **H17** undergoes hydrogenation catalyzed by palladium carbon to remove the benzyl group to obtain the target product **H18.** Wherein, n is 1-10; X is oxygen, sulfur, and nitrogen; R is C1-C8 alkyl.

In one embodiment, the compound or a pharmaceutically acceptable salt thereof is prepared by the following route:

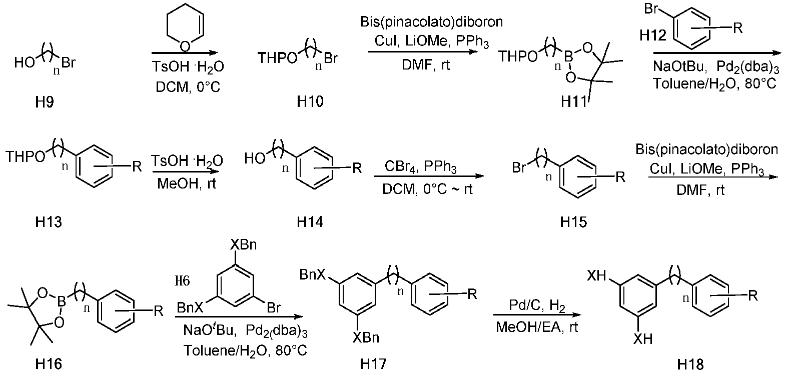

Compounds of the present invention with other different substituents can be prepared by the following specific methods:

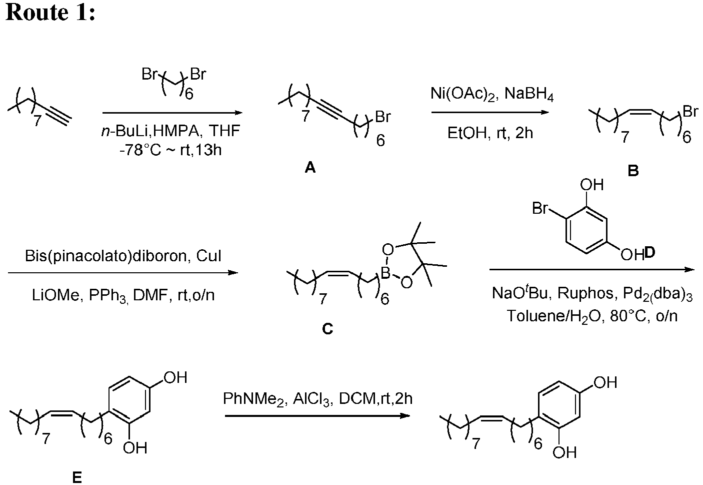

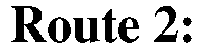

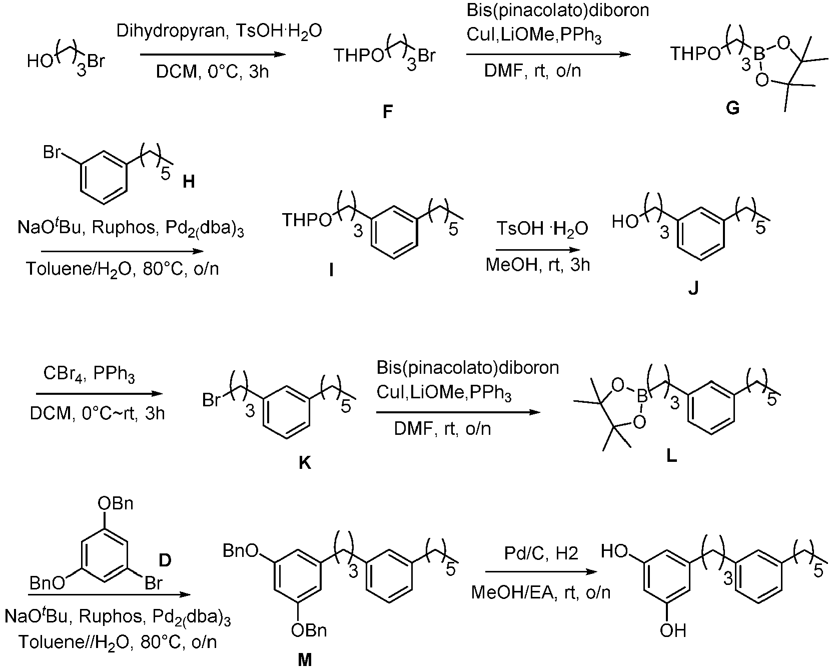

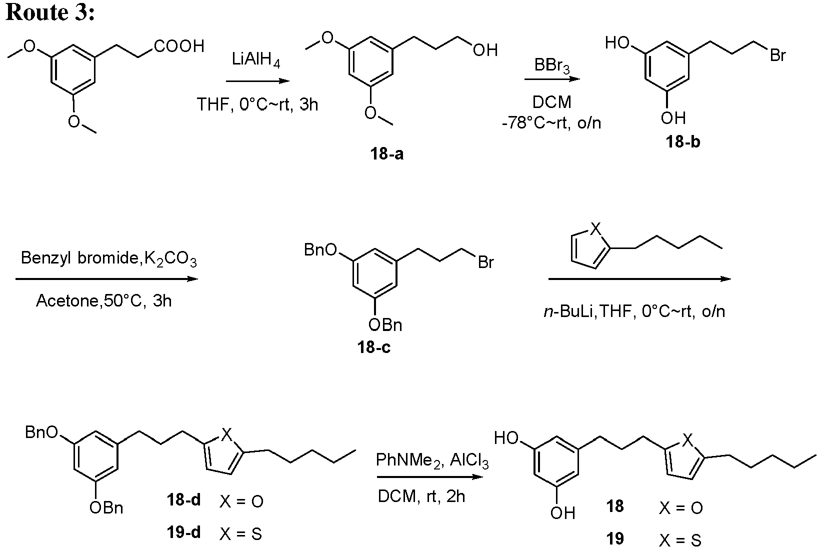

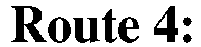

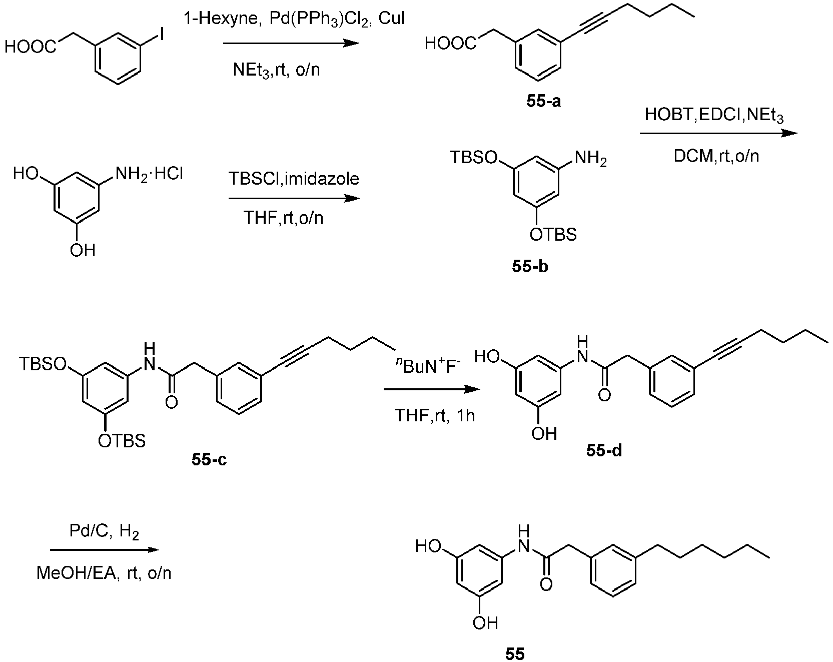

### Pharmaceutical composition and mode of administration

Due to the excellent kinase inhibitory activity of the compounds of the present invention, the compounds of the invention and various crystal forms, pharmaceutically acceptable inorganic or organic salts, hydrates or solvates thereof, and pharmaceutical compositions containing the compound of the present invention as an active ingredient can be used in the treatment, prevention and alleviation of the related diseases induced by the abnormal expression or activity of kinase(such as DRAK2).

The pharmaceutical composition of the present invention comprises a safe and effective amount of the compound of the present invention, or a pharmaceutically acceptable salt thereof and pharmaceutically acceptable excipients or carriers. Wherein the "safe and effective amount" refers to the amount of compound which is sufficient to significantly improve the condition, and not to generate severe side effects. Generally, the pharmaceutical composition contains 1-2000 mg polymorphs of the invention per dose, preferably, 5-200mg polymorphs of the invention per dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatible" herein refers to the ability of each component of a composition can be mixed with the compound of the present invention and can be mixed with each other without appreciably reducing the efficacy of the compound. Examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween^{®}), wetting agent (such as lauryl sodium sulfate), colorant, flavoring, stabilizer, antioxidant, preservative, pyrogen-free water, etc.

There is no special limitation of administration mode for the compound or pharmaceutical compositions of the present invention, and the representative administration mode includes (but is not limited to): oral, intratumoral, rectal, parenteral (intravenous, intramuscular or subcutaneous), and topical administration.

The solid dosage forms used for oral administration include capsules, tablets, pills, powders, and granules. In these solid dosage forms, the active compounds are mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets, and pills, the dosage form may also include buffers.

Solid dosage forms such as tablets, sugar pills, capsules, pills, and granules can be prepared using coating and shell materials, such as casings and other materials well-known in the art. They can contain opacifiers, and the release of active compounds or compounds in the composition can be delayed in a certain part of the digestive tract. Examples of embedding components that may be employed are polymeric substances and waxes. If necessary, the active compound may also form a microcapsule with one or more of the above excipients.

Liquid dosage forms for oral administration include pharmaceutically acceptable lotion, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

In addition to these inert diluents, the composition can also include additives such as wetting agents, emulsifiers and suspensions, sweeteners, corrigens, and flavouring agent.

In addition to active compounds, suspensions can include suspending agents such as ethoxylatedisooctadecanol, polyoxyethylene sorbitol and dehydrated sorbitol esters, microcrystalline cellulose, methanol aluminum and agar, or mixtures thereof.

Compositions for parenteral injection may include physiologically acceptable sterile aqueous or anhydrous solutions, dispersion liquid, suspensions or lotions, and sterile powders for re dissolution into sterile injectable solutions or dispersions. Suitable aqueous and non-aqueous carriers, diluents, solvents or excipients include water, ethanol, polyols, and their suitable mixtures.

The dosage forms of the compounds of the present invention used for local administration include ointments, powders, patches, sprays, and inhalants. The active ingredients are mixed under sterile conditions with physiologically acceptable carriers and any preservatives, buffers, and propellants if necessary.

The compounds of the present invention may be administered alone or in combination with other pharmaceutically acceptable compound. In some preferred embodiments, the compounds of the present invention can form PROTAC with other small molecule compounds, or jointly form ADC with other large molecule compounds such as monoclonal antibodies for application.

When the pharmaceutical compositions are used, a safe and effective amount of compound of the present invention is applied to a mammal (such as human) in need of, wherein the dose of administration is a pharmaceutically effective dose. For a person weighed 60 kg, the daily dose is usually 1- 2000 mg, preferably 5-500 mg. Of course, the particular dose should also depend on various factors, such as the route of administration, patient healthy status, which are well within the skills of an experienced physician.

**Compared with the prior art, the main advantages of the present invention include:**
The compound of the present invention can effectively inhibit DRAK2 kinase, with a DRAK2 inhibitory activity at micro molar concentrations. The compound of the present invention has a simple and novel structure, and can be easily synthesized and prepared, and has better oral absorption.

The present invention was further described hereafter in combination with specific embodiments. It should be understood that these examples are only used to illustrate the and not to limit the scope of the invention. The experimental methods with no specific conditions described in the following examples are generally performed under the conventional conditions, for example, according to J. Sambrook et al., Molecular Cloning: A Laboratory Manual, 3rd Edition, Science Press, 1989, or according to the manufacture's instructions. Unless otherwise stated, percentages and parts are caculated by weight.

Unless otherwise defined, all professional and scientific terms used herein have the same meanings as those familiar to those skilled in the art. In addition, any methods and materials similar or equal to the recorded content can be applied to the methods of the present invention. The preferred embodiments and materials described herein are for exemplary purposes only.

In the following preparation examples, NMR was measured using the Mercury Vx 400M instrument produced by Varian, and NMR was calibrated as follows: δ H7.26 ppm (CDCl₃), 2.50 ppm (DMSO-d6); Mass spectrometry was determined using Agilent 1200Quadrupole LC/MS liquid chromatography-mass spectrometry or SHIMADZU GCMS-QP5050A; The reagents are mainly provided by Shanghai Chemical Reagent Company; TLC thin layer chromatography silica gel plate is produced by Shandong Yantai Huiyou Silicone Development Co., Ltd., model HSGF 254; The normal phase column chromatography silica gel used for compound purification is produced by Shandong Qingdao Ocean Chemical Factory Branch, with the model of zcx-1, a mesh of 1200-300.

The names corresponding to the abbreviations used herein are as follows:
DMAP: 4-Dimethylaminopyridine; DCM: dichloromethane; DMF: N, N-dimethylformamide; HOBT: 1-hydroxybenzotriazole; MeOH: Methanol; THF: Tetrahydrofuran; TBSCl: tert butyl dimethyl chlorosilane

### Example 1

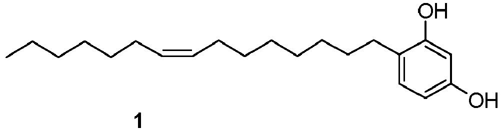

To a solution of alkyne compound(4.54mmol) in tetrahydrofuran was dropwise added n-butyl lithium (1.89 mL, 2.4 M in hexane, 4.54 mmol) at -78 °C, and the reaction solution was gradually warmed to room temperature for 30 minutes. Then cooling to -78 ° C, hexamethylphosphorictriamide (0.79 mL, 4.54 mmol) and a solution of dibromoalkane (3.78 mmol) in tetrahydrofuran (5 mL) were added successively. After addition, the reaction solution was gradually warmed to room temperature, and stirred for 2 hours. After the reaction was completed, the reaction was quenched with NH₄Cl saturated solution (10 mL), and extracted with ethyl acetate (3 × 10 mL). The combined organic phase was washed with saturated salt water (10 mL), dried with anhydrous sodium sulfate, concentrated with a rotary evaporator to remove solvent, and purified with column chromatography (petroleum ether) to obtain intermediate compound A. ¹H NMR (400 MHz, Chloroform-d) δ 3.40 (t, J = 8.0 Hz, 2H), 2.23-2.05 (m, 4H), 1.88-1.84 (m, 2H), 1.53-1.21 (m, 16H), 0.89 (t, J = 6.8 Hz, 3H).

To a solution of Nickel acetate (93.74 mg, 0.378 mmol) in 2 mL of anhydrous ethanol , sodium borohydride (14.36 mg, 0.378 mmol) was added, and degassed with nitrogen for three times, and then degassed with hydrogen for three times. After stirring at room temperature for 15 minutes, ethylenediamine (0.1 mL, 1.51 mmol) was added, and reacted for 15 minutes, then a solution of intermediate A (0.775 mmol) in 1 mL of anhydrous ethanol was added dropwise. The resulting solution was stirred at room temperature for 2 hours, and tracked and detected with TLC. After the reaction was completed, the reaction solution was filtered with diatomite. The filter residue was washed with petroleum ether, the filtrate was concentrated, and finally isolated by column chromatography purification (petroleum ether) to obtain intermediate B. ¹H NMR (400 MHz, Chloroform-d) δ 5.42-5.27 (m, 2H), 3.40 (t, J = 7.0 Hz, 2H), 2.10-1.93 (m, 4H), 1.85 (t, J = 7.3 Hz, 2H), 1.46-1.39 (m, 2H), 1.37-1.24 (m, 14H), 0.89 (t, J = 6.0 Hz, 3H).

To a three-neck bottle was added successively copper iodide (4.8 mg, 0.025 mmol), triphenylphosphine(8.6 mg, 0.033 mmol), lithium methoxide (20 mg, 0.5 mmol), and bis(pinacolato)diboron (96.5 mg, 0.38 mmol). After addition, the mixture was degassed with N₂ for three times, then a solution of intermediate B (73 mg, 0.25 mmol) in N, N-dimethylformamide was added. The reaction solution was stirred at room temperature for 18 hours and traced and detected with TLC. After the reaction was completed, the reaction solution was diluted with ethyl acetate, and filtered with diatomite to remove solid. The filter residue was washed with ethyl acetate, and the filtrate was concentrated and finally separated by column chromatography purification [eluent:V(petroleum ether):V(ethyl acetate) = 20:1] to obtain intermediate C. ¹H NMR (400 MHz, Chloroform-d) δ 5.31-5.25 (m, 2H), 2.02-1.86 (m, 4H), 1.29-1.17 (m, 30H), 0.81 (t, J = 6.2 Hz, 3H), 0.70 (t, J = 8.0 Hz, 2H).

To a solution of bromohydroquinone or bromothyrogallol (2.64 mmol) in 20 mL of acetone was added potassium carbonate (547 mg, 4 mmol), and benzyl bromide (0.72 mL, 6.07 mmol) successively. The reaction solution was stirred overnight at 50 °C, and traced by TLC detection. After the reaction was completed, the reaction solution was cooled to room temperature and filtered with diatomite to remove solid. The filter residue was washed with dichloromethane, and the filtrate was concentrated, and finally separated by column chromatography purification[eluent:V(petroleum ether):V(ethyl acetate) = 20:1]to obtain intermediate D. ¹H NMR (400 MHz, Chloroform-d) δ 7.47-7.30 (m, 10H), 6.78-6.75 (m, 2H), 6.54 (s, 1H), 5.03-4.98 (m, 4H).

To a three-neck bottle was added successively intermediate C (0.271 mmol), sodium tert butanol (78.1 mg, 0.813 mmol), Ruphos (31 mg, 0.0664 mmol), Pd₂ (dba)₃ (30 mg, 0.0328 mmol), and intermediate D (0.325 mmol). After addition, the mixture was degassed with N₂ for multiple times, and toluene (3 mL) and 0.3 mL of deionized water were added successively. The reaction solution was heated to 80°C, stirred for 18 hours and traced and detected with TLC. After the reaction was completed, the reaction solution was diluted with ethyl acetate, and filtered with diatomite to remove solid. The filter residue was washed with ethyl acetate, and the filtrate was concentrated and finally separated by column chromatography purification [eluent:V(petroleum ether):V(ethyl acetate) = 20:1] to obtain intermediate E. ¹H NMR (400 MHz, Chloroform-d) δ 7.37-7.23 (m, 10H), 6.41-6.34 (m, 3H), 5.32 -5.22 (m, 2H), 4.98 -4.91 (m, 4H), 2.46 (t, J = 7.8 Hz, 2H), 1.99-1.89 (m, 4H), 1.56-1.47 (m, 2H), 1.29-1.18 (m, 16H), 0.83-0.78 (m, 3H).

To a solution of intermediate E (0.144 mmol) in 4 mL of dichloromethane was added N, N-dimethylaniline (0.12 mL, 0.938 mmol) and aluminum trichloride (115.2 mg, 0.864 mmol), then reacted at room temperature for 2 hours, and traced and detected with TLC. After the reaction was completed, the reaction solution was diluted with 5 mL of dichloromethane, then poured into 5 mL of 1N HCL solution and stirred for 5 minutes. The oil phase was separated and the aqueous phase was extracted with dichloromethane (2 × 5 mL). The combined organic phase was washed with 10 mL saturated salt water, dried with anhydrous sodium sulfate, concentrated to remove solvent, and purified by column chromatography to obtain final product 1. ¹H NMR (400 MHz, Chloroform-d) δ 6.26 -6.22 (m, 2H), 6.17 (s, 1H), 5.37-5.32 (m, 2H), 4.80-4.65 (m, 2H), 2.48 (t, J = 7.8 Hz, 2H), 2.05-1.97 (m, 4H), 1.66 (t, J = 7.2 Hz, 2H), 1.33-1.26 (m, 16H), 0.87 (t, J = 7.2 Hz, 3H).

The following compounds were synthesized using the same method as in Example 1

| Compo und No. | Structure | NMR |
|---|---|---|
| **2** | 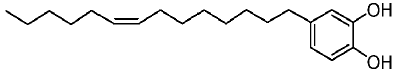 | ¹H NMR (400 MHz, Chloroform-d) δ 6.76 (d, J = 8.0 Hz, 1H), 6.70 (s, 1H), 6.60 (d, J = 8.0 Hz, 1H), 5.39-5.28 (m, 2H), 5.25-4.99 (m, 2H), 2.48 (t, J = 7.8 Hz, 2H), 2.10-1.93 (m, 4H), 1.55 (t, J = 7.4 Hz, 2H), 1.34-1.24 (m, 16H), 0.89 (t, J = 7.8 Hz ,3H). |
| **3** | 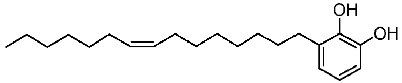 | ¹H NMR (400 MHz, Chloroform-d) δ 6.75 -6.66 (m, 3H), 5.43-5.27 (m, 2H), 5.17-5.00 (m, 2H), 2.60 (t, *J* = 7.9 Hz, 2H), 2.08-1.92 (m, 2H), 1.67-1.55 (m, 4H), 1.40-1.22 (m, 16H), 0.88 (t, *J* = 6.5 Hz, 3H). |
| **4** | 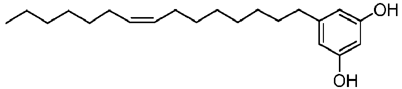 | ¹H NMR (400 MHz, Chloroform-d) δ 6.27 -6.21 (m, 2H), 6.17 (s, 1H), 5.43-5.26 (m, 2H), 4.95-4.74 (m, 2H), 2.48 (t, *J* = 7.9 Hz, 2H), 2.09-1.95 (m, 4H), 1.63-1.51 (m, 2H), 1.35-1.26 (m, 16H), 0.93-0.85 (m, 3H). |
| **5** | 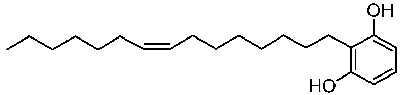 | ¹H NMR (400 MHz, Chloroform-d) δ 6.91 (t, *J* = 8.1 Hz, 1H), 6.43-6.34 (m, 2H), 5.40-5.29 (m, 2H), 4.86-4.58 (m, 2H), 2.62 (t, *J* = 7.8 Hz, 2H), 2.07-1.93 (m, 4H), 1.60-1.53 (m, 2H), 1.37-1.23 (m, 16H), 0.88 (t, *J* = 7.2 Hz, 3H). |
| **6** | 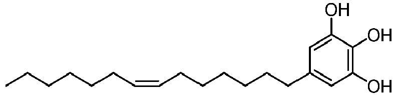 | ¹H NMR (400 MHz, Chloroform-d) δ 6.34 -6.25 (m, 2H), 5.62-5.26 (m, 5H), 2.41 (t, *J* = 7.8 Hz, 2H), 2.04-1.97 (m, 4H), 1.56-1.48 (m, 2H), 1.34-1.25 (m, 14H), 0.89-0.85 (m, 3H). |
| **9** | 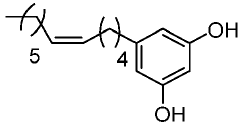 | ¹H NMR (400 MHz, Chloroform-d) δ 6.27 -6.22 (m, 2H), 6.18 (s, 1H), 5.41-5.28 (m, 2H), 5.08-4.99 (m, 2H), 2.54-2.41 (m, 2H), 2.07-1.98 (m, 4H), 1.64-1.53 (m, 2H), 1.39-1.22 (m, 10H), 0.88 (t, J = 5.6 Hz, 3H). |
| **10** | 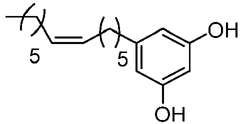 | ¹H NMR (400 MHz, Chloroform-d) δ 6.26-6.22 (m, 2H), 6.17 (d, J = 2.4 Hz, 1H), 5.37-5.32 (m, 2H), 4.67-4.64 (m, 2H), 2.49 (t, J = 7.5 Hz, 2H), 2.07-1.97 (m, 4H), 1.63-1.57 (m, 2H), 1.38-1.26 (m, 12H), 0.88 (t, J = 6.4 Hz, 3H). |
| **11** | 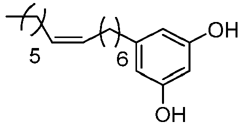 | ¹H NMR (400 MHz, Chloroform-d) δ 6.26 -6.21 (m, 2H), 6.17 (s, 1H), 5.34 (m, 2H), 4.66 -4.62 (m, 2H), 2.50 (t, J = 9.1 Hz, 2H), 2.06-1.97 (m, 4H), 1.36-1.24 (m, 16H), 0.88 (t, J = 5.5 Hz, 3H). |
| **12** | 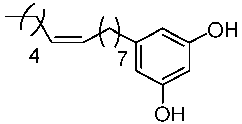 | ¹H NMR (400 MHz, Chloroform-d) δ 6.26-6.22 (m, 2H), 6.17 (s, 1H), 5.37-5.32 (m, 2H), 4.66-4.62 (m, 2H), 2.49 (t, J = 8.8 Hz, 2H), 2.07-1.97 (m, 4H), 1.44-1.17 (m, 16H), 0.89 (t, J = 7.1 Hz, 3H). |
| **13** | 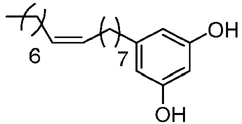 | ¹H NMR (400 MHz, Chloroform-d) δ 6.26-6.20 (m, 2H), 6.17 (s, 1H), 5.39-5.30 (m, 2H), 4.65-4.61 (m, 2H), 2.49 (t, J = 9.0 Hz, 2H), 2.05-1.97 (m, 4H), 1.37-1.18 (m, 20H), 0.91-0.86 (m, 3H). |
| **14** | 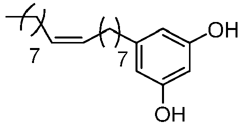 | ¹H NMR (400 MHz, Chloroform-d) δ 6.28- 6.22 (m, 2H), 6.17 (s, 1H), 5.44-5.08 (m, 4H), 2.47 (t, J = 9.3 Hz, 2H), 2.01 (t, J = 5.7 Hz, 4H), 1.56 (t, J = 10.2 Hz, 2H), 1.36 - 1.26 (m, 20H), 0.88 (t, J = 5.6 Hz, 3H). |
| **15** | 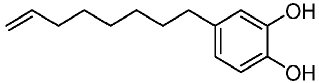 | ¹H NMR (400 MHz, Chloroform-d) δ 6.76 (d, J = 8.1 Hz, 1H), 6.70 (s, 1H), 6.60 (d, J = 8.1 Hz, 1H), 5.80 (td, J = 10.1, 4.8 Hz, 1H), 5.13-4.87 (m, 4H), 2.49 (t, J = 7.7 Hz, 2H), 2.02 (t, J = 7.1 Hz, 2H), 1.57-1.52 (m, 2H), 1.42-1.27 (m, 6H) |
| **26** | 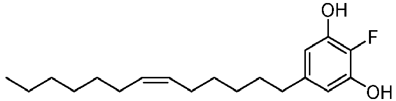 | ¹H NMR (400 MHz, Chloroform-d) δ 6.41 -6.32 (m, 2H), 5.47-5.01 (m, 4H), 2.45 (t, J = 7.8 Hz, 2H), 2.08-1.97 (m, 4H), 1.55 (p, J = 7.5 Hz, 2H), 1.38-1.22 (m, 14H), 0.88 (t, J = 5.6 Hz, 3H). |

### Example 2

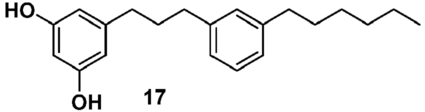

To a solution of halohydrin(11.36 mmol) in 12 mL of dichloromethane was added successively 3,4-2H-dihydropyran (1.1 mL, 12.17 mmol) and p-toluenesulfonic acid hydrate (10 mg, 0.0526 mmol) at 0 °C, then reacted at room temperature for 2 hours, and traced and detected with TLC. After the reaction was completed, the reaction solution was directly concentrated to remove solvent, and finally separated and purified by column chromatography [eluent: V (petroleum ether): V (ethyl acetate)=20:1] to obtain intermediate F. ¹H NMR (400 MHz, Chloroform-d) δ 4.60 (dd, J = 4.4, 2.8 Hz, 1H), 3.92-3.81 (m, 2H), 3.58-3.49 (m, 4H), 2.18-2.09 (m, 2H), 1.88-1.76 (m, 1H), 1.76-1.65 (m, 1H), 1.61-1.51 (m, 4H).

To a three-neck bottle was added successively copper iodide (26 mg, 0.134 mmol), triphenylphosphine(45 mg, 0.174 mmol), lithium methoxide (102 mg, 2.68 mmol), and bis(pinacolato)diboron (510 mg, 2.01 mmol), then degassed with N₂ for three times, and a solution of intermediate F (1.34 mmol) in N, N-dimethylformamide(3.0 mL) was added. The reaction solution was stirred at room temperature for 18 hours and traced and detected with TLC. After the reaction was completed, the reaction solution was diluted with ethyl acetate, and filtered with diatomite to remove solid. The filter residue was washed with ethyl acetate, and the filtrate was concentrated and finally separated by column chromatography[eluent:V(petroleum ether):V(ethyl acetate) = 20:1] to obtain intermediate G. ¹H NMR (400 MHz, Chloroform-d) δ 4.59 (q, J = 3.9 Hz, 1H), 3.86 (td, J = 8.3, 4.2 Hz, 1H), 3.68 (ddd, J = 9.5, 6.7, 2.6 Hz, 1H), 3.55-3.42 (m, 1H), 3.42-3.29 (m, 1H), 1.92-1.77 (m, 1H), 1.70 (q, J = 7.7 Hz, 3H), 1.63-1.45 (m, 4H), 1.32-1.15 (m, 12H), 0.90-0.74 (m, 2H).

To a three-neck bottle was added successively intermediate C (0.271 mmol), sodium tert butanol (78.1 mg, 0.813 mmol), Ruphos (31 mg, 0.0664 mmol), Pd₂ (dba)₃ (30 mg, 0.0328 mmol), and intermediate G (0.325 mmol), then degassed with N₂ for multiple times, and toluene (3 mL) and deionized water(0.3 mL) was added successively. The reaction solution was heated to 80°C, stirred for 18 hours and traced and detected with TLC. After the reaction was completed, the reaction solution was diluted with ethyl acetate, and filtered with diatomite to remove solid. The filter residue was washed with ethyl acetate, and the filtrate was concentrated and finally separated and purified by column chromatography [eluent:V(petroleum ether):V(ethyl acetate) = 20:1] to obtain intermediate I. ¹H NMR (400 MHz, Chloroform-d) δ 7.17 (t, J = 7.6 Hz, 1H), 7.00 (d, J = 6.4 Hz, 3H), 4.57 (t, J = 3.8 Hz, 1H), 3.87 (t, J = 9.6 Hz, 1H), 3.77 (q, J = 7.5 Hz, 1H), 3.54-3.44 (m, 1H), 3.40 (q, J = 7.5 Hz, 1H), 2.68 (q, J = 6.6 Hz, 2H), 2.56 (t, J = 7.9 Hz, 2H), 1.97-1.78 (m, 3H), 1.77-1.66 (m, 1H), 1.65-1.46 (m, 6H), 1.39-1.22 (m, 6H), 0.87 (t, J = 6.8 Hz, 3H).

To a solution of intermediate I (2.25 mmol) in methanol (10 mL) was added p-toluenesulfonic acid hydrate (20 mg, 0.05 mmol), then stirred at room temperature for 3 hours and traced and detected with TLC. After the reaction was completed, the reaction solution was concentrated to remove solvent, then separated and purified by column chromatography [eluent: V (petroleum ether): V (ethyl acetate)=5:1] to obtain intermediate J. 1H NMR (400 MHz, Chloroform-d) δ 7.18 (t, J = 7.6 Hz, 1H), 7.03-6.97 (m, 3H), 3.66 (t, J = 7.6 Hz, 2H), 2.67 (t, J = 7.9 Hz, 2H), 2.57 (t, J = 7.9 Hz, 2H), 1.89 (q, J = 7.2 Hz, 2H), 1.59 (q, J = 7.5 Hz, 2H), 1.50 (s, 1H), 1.39-1.24 (m, 6H), 0.87 (t, J = 6.9 Hz, 3H).

Intermediate J (1.41 mmol) and carbon tetrabromide (701 mg, 2.1 mmol) were dissolved in 3 mL of dichloromethane and cooled to 0 °C, and a solution of triphenylphosphine (550 mg, 2.1 mmol) in dichloromethane (1 mL) was added dropwise. The reaction solution was warmed to room temperature and reacted for 3 hours, and traced detected with TLC. After the reaction was completed, the reaction solution was concentrated to remove solvent, then separated and purified by column chromatography (petroleum ether) to obtain intermediate K. ¹H NMR (400 MHz, Chloroform-d) δ 7.25-7.15 (m, 1H), 7.05 - 6.96 (m, 3H), 3.39 (t, J = 6.6 Hz, 2H), 2.74 (t, J = 7.4 Hz, 2H), 2.57 (t, J = 7.9 Hz, 2H), 2.16 (p, J = 7.0 Hz, 2H), 1.59 (q, J = 7.5 Hz, 2H), 1.39-1.22 (m, 6H), 0.88 (t, J = 6.6 Hz, 3H).

To a three-neck bottle was added successively copper iodide (26 mg, 0.134 mmol), triphenylphosphine (45 mg, 0.174 mmol), lithium methoxide (102 mg, 2.68 mmol), and bis(pinacolato)diboron (510 mg, 2.01 mmol), then degassed with N₂ for three times, and added with a solution of intermediate K (1.34 mmol) in N, N-dimethylformamide(3.0 mL). The reaction solution was stirred at room temperature for 18 hours and traced and detected with TLC. After the reaction was completed, the reaction solution was diluted with ethyl acetate, and filtered with diatomite to remove solid. The filter residue was washed with ethyl acetate, and the filtrate was concentrated and finally separated by column chromatography[eluent:V(petroleum ether):V(ethyl acetate) = 20:1] to obtain intermediate L. ¹H NMR (400 MHz, Chloroform-d) δ 7.16 (t, J = 7.7 Hz, 1H), 7.01- 6.94 (m, 3H), 2.63-2.50 (m, 4H), 1.72 (t, J = 7.9 Hz, 2H), 1.62-1.51 (m, 2H), 1.41-1.15 (m, 18H), 0.93-0.77 (m, 5H).

To a three-neck bottle was added successively intermediate D (0.271 mmol), sodium tert butanol (78.1 mg, 0.813 mmol), Ruphos (31 mg, 0.0664 mmol), Pd₂ (dba)₃ (30 mg, 0.0328 mmol), and intermediate L (0.325 mmol), then degassed with N₂ for multiple times, and toluene (3 mL) and deionized water(0.3 mL) were added successively. The reaction solution was heated to 80°C, stirred for 18 hours and traced and detected with TLC. After the reaction was completed, the reaction solution was diluted with ethyl acetate, and filtered with diatomite to remove solid. The filter residue was washed with ethyl acetate, and the filtrate was concentrated and finally separated and purified by column chromatography [eluent:V(petroleum ether):V(ethyl acetate) = 20:1] to obtain intermediate M. ¹H NMR (400 MHz, Chloroform-d) δ 7.48-7.29 (m, 10H), 7.20 (s, 1H), 7.05-6.96 (m, 3H), 6.52-6.44 (m, 3H), 5.07-5.00 (m, 4H), 2.73-2.49 (m, 6H), 2.02-1.89 (m, 2H), 1.67 -1.58 (m, 2H), 1.35-1.29 (m, 6H), 0.89 (t, J = 7.2Hz,3H).

To a solution of the intermediate protected by benzyl group (0.2 mmol) in a mixed solvent of 2 mL EA and 2 mL MeOH was added palladium carbon, then degassed with N₂ for multiple times, and degassed with H₂ for multiple times, and then reacted overnight at room temperature. After the reaction was completed, the reaction solution was filtered with diatomite, and the filtrate was concentrated, then separated by column chromatography[eluent: V (dichloromethane): V (methanol)=20:1] to obtain final product 17. 1H NMR (400 MHz, Chloroform-d) δ 7.20 -7.16 (m, 1H), 7.03-6.96 (m, 3H), 6.28-6.22 (m, 2H), 6.18 (s, 1H), 2.62-2.50 (m, 6H), 1.95-1.87 (m, 2H), 1.64-1.56 (m, 2H), 1.34-1.28 (m, 6H), 0.88 (t, J = 6.8 Hz, 3H).

The following compounds were synthesized using the same method as in Example 2.

| Compo und No. | Structure | NMR |
|---|---|---|
| **16** | 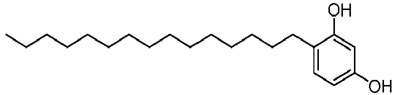 | ¹H NMR (400 MHz, Chloroform-d) δ 6.26-6.22 (m, 2H), 6.17 (s, 1H), 4.81-4.75 (m, 2H), 2.48 (t, J = 7.8 Hz, 2H), 1.56-1.52(m, 2H), 1.29-1.22 (m, 24H), 0.88 (t, J = 6.4 Hz, 3H). |
| **20** | 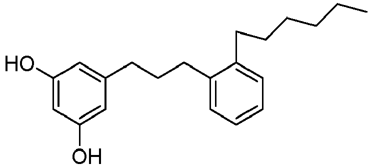 | ¹H NMR (400 MHz, Chloroform-d) δ 7.15 -7.09 (m, 4H), 6.27-6.25 (m, 2H), 6.18 (s, 1H), 4.70-4.72 (m, 2H), 2.65-2.53 (m, 6H), 1.87-1.84 (m, 2H), 1.55-1.48 (m, 2H), 1.34-1.28 (m, 6H), 0.89 (t, J = 6.8 Hz, 3H). |
| **21** | 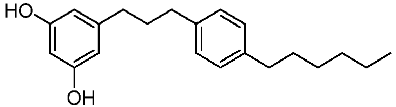 | ¹H NMR (400 MHz, Chloroform-d) δ 7.10-7.00 (m, 4H), 6.25-6.19 (m, 2H), 6.15 (s, 1H), 6.10-5.90 (m, 2H), 2.61-2.49 (m, 4H), 2.44 (t, *J* = 7.9 Hz, 2H), 1.83-1.80 (m, 2H), 1.63-1.51 (m, 2H), 1.34-1.25 (m, 6H), 0.87 (t, *J* = 6.8 Hz, 3H). |
| **27** | 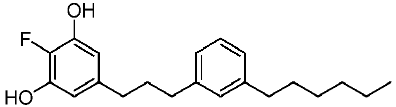 | ¹H NMR (400 MHz, Chloroform-d) δ 7.18 (t, *J* = 7.7 Hz, 1H), 7.02-6.95 (m, 3H), 6.41-6.33 (m, 2H), 5.76-4.98 (m, 2H), 2.64 -2.46 (m, 6H), 1.93-1.82 (m, 2H), 1.61-1.58 (m, 2H), 1.36-1.28 (m, 6H), 0.88 (d, *J* = 5.6 Hz, 3H). |
| **29** | 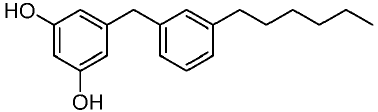 | ¹H NMR (400 MHz, Chloroform-d) δ 7.19 (t, *J* = 7.6 Hz, 1H), 7.06-6.95 (m, 3H), 6.25-6.21 (m, 2H), 6.18 (s, 1H), 5.16-4.37 (m, 2H), 3.82 (s, 2H), 2.56 (t, *J* = 7.9 Hz, 2H), 1.64-1.54 (m, 2H), 1.37-1.24 (m, 6H), 0.87 (t, *J* = 6.8 Hz, 3H). |
| **30** | 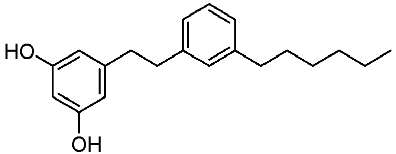 | ¹H NMR (400 MHz, Chloroform-d) δ 7.19 (t, J = 7.5 Hz, 1H), 7.03-6.97 (m, 3H), 6.26-6.25 (m, 2H), 6.19 (t, J = 2.2 Hz, 1H), 4.75 (m, 2H), 2.89-2.76 (m, 4H), 2.57 (dd, J = 8.9, 6.8 Hz, 2H), 1.64-1.61 (m, 2H), 1.36 -1.28 (m, 6H), 0.89 (t, J = 5.2 Hz, 3H). |
| **31** | 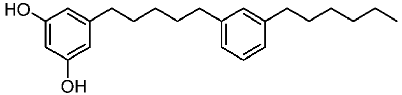 | ¹H NMR (400 MHz, Chloroform-d) δ 7.18 (t, *J* = 7.6 Hz, 1H), 7.04-6.94 (m, 3H), 6.27-6.10 (m, 3H), 5.55-4.83 (m, 2H), 2.62 -2.52 (m, 4H), 2.46 (t, *J* = 7.9 Hz, 2H), 1.66-1.53 (m, 6H), 1.39-1.26 (m, 8H), 0.88 (t, *J* = 6.8 Hz, 3H). |
| **32** | 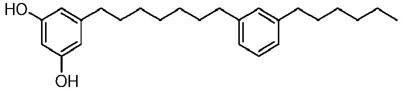 | ¹H NMR (400 MHz, Chloroform-d) δ 7.16 (t, *J* = 7.6 Hz, 1H), 7.01-6.95 (m, 3H), 6.29-6.12 (m, 3H), 5.16-4.16 (m, 2H), 2.63 -2.51 (m, 4H), 2.46 (t, *J* = 7.9 Hz, 2H), 1.65-1.51 (m, 6H), 1.35-1.25 (m, 12H), 0.88 (t, *J* = 6.8 Hz, 3H). |
| **33** | 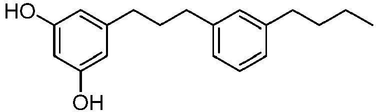 | ¹H NMR (400 MHz, Chloroform-d) δ 7.18 (t, J = 7.7 Hz, 1H), 7.04-6.95 (m, 3H), 6.29-6.21 (m, 2H), 6.18 (t, J = 2.3 Hz, 1H), 4.77-4.69 (m, 2H), 2.66-2.48 (m, 6H), 1.96-1.86 (m, 2H), 1.64-1.59 (m, 2H), 1.42-1.30 (m, 2H), 0.93 (t, J = 7.3 Hz, 3H). |
| **34** | 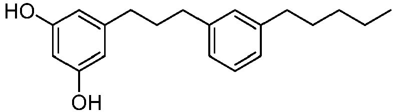 | ¹H NMR (400 MHz, Chloroform-d) δ 7.17 (t, *J* = 7.5 Hz, 1H), 7.02-6.94 (m, 3H), 6.24-6.23 (m, 2H), 6.22-6.12 (m, 1H), 5.55 -5.25 (m, 2H), 2.63-2.45 (m, 6H), 1.88 (t, *J* = 7.8 Hz, 2H), 1.67-1.53 (m, 2H), 1.39-1.28 (m, 4H), 0.88 (t, *J* = 6.8 Hz, 3H). |
| **35** | 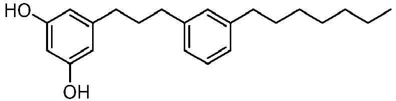 | ¹H NMR (400 MHz, Chloroform-d) δ 7.18 (t, J = 7.7 Hz, 1H), 7.02-6.97 (m, 3H), 6.26-6.24 (m, 2H), 6.19-6.17 (m, 1H), 4.74 -4.70 (m, 2H), 2.63-2.51 (m, 6H), 1.97-1.86 (m, 2H), 1.64-1.59 (m, 2H), 1.35-1.25 (m, 8H), 0.88 (t, J = 6.7 Hz, 3H). |
| **36** | 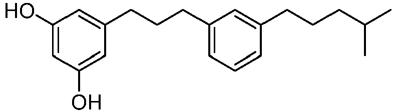 | ¹H NMR (400 MHz, Chloroform-d) δ 7.19 (t, *J* = 7.7 Hz, 1H), 6.97-7.03 (m, 3H), 6.27-6.24 (m, 2H), 6.18 (t, *J* = 2.3 Hz, 1H), 4.76-4.70 (m, 2H), 2.65-2.48 (m, 6H), 1.94-1.90 (m, 2H), 1.63-1.59 (m, 2H), 1.28-1.18 (m, 3H), 0.90-0.85 (m, 6H). |
| **37** | 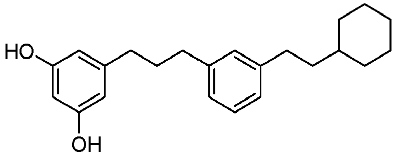 | ¹H NMR (400 MHz, Chloroform-d) δ 7.18 (t, J = 7.7 Hz, 1H), 7.03-6.96 (m, 3H), 6.26-6.23 (m, 2H), 6.18 (t, J = 2.3 Hz, 1H), 4.96-4.91 (m, 2H), 2.66-2.50 (m, 6H), 1.94-1.87 (m, 2H), 1.80-1.72 (m, 2H), 1.55-1.44 (m, 2H), 1.29-0.83 (m, 9H). |
| **38** | 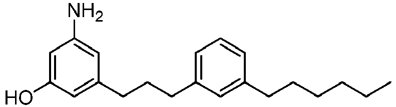 | ¹H NMR (400 MHz, Chloroform-d) δ 7.18 (s, 1H), 7.02-6.97 (m, 3H), 6.12 (dd, J = 1.7, 1.8 Hz, 1H), 6.08 (dd, J = 1.8, 2.2 Hz, 1H), 6.02 (dd, J = 1.7, 2.2 Hz, 1H), 4.57 (s, 1H), 3.61 (s, 2H), 2.64-2.54 (m, 4H), 2.50 (t, J = 8.0 Hz, 2H), 1.95-1.86 (m, 2H), 1.62-1.58 (m, 2H), 1.37-1.27 (m, 6H), 0.88 (t, J = 5.6 Hz, 3H). |
| **56** | 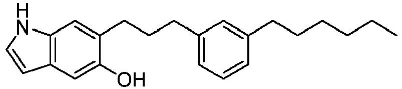 | ¹H NMR (400 MHz, Chloroform-d) δ 7.76 (s, 1H), 7.24-7.18 (m, 1H), 7.10 (dd, *J* = 3.1,2.3 Hz, 1H), 7.05-7.00 (m, 3H), 6.89 (d, *J* = 2.3 Hz, 1H), 6.62 (d, *J* = 2.3 Hz, 1H), 6.41 (dd, *J* = 3.1, 2.0 Hz, 1H), 4.47 (s, 1H), 2.78 (t, *J* = 7.6 Hz, 2H), 2.69 (t, *J* = 7.6 Hz, 2H), 2.58 (t, *J* = 7.6 Hz, 2H), 2.11-2.02 (m, 2H), 1.64-1.58 (m, 2H), 1.35-1.27 (m, 6H), 0.86 (t, 5.6Hz, 3H). |

### Example 3

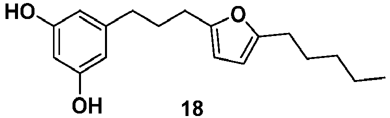

To a solution of lithium aluminum hydride (1.1 g, 28.54 mmol) in 20 mL of re-distilled THF was slowly added a solution of 3,5-dimethoxyphenylpropionic acid (2.0 g, 9.51 mmol) in 10 mL of THF at 0 °C, then gradually warmed to room temperature, reacted for 3 hours, and traced and detected with TLC. After the reaction was completed, the reaction solution was diluted with 20 mL of ether and quenched with 2 mL of slowly added water with gray white solid generated. The mixture was alkalized with 50 mL of 15% sodium hydroxide solution, and fractioned. The aqueous phase was extracted with ether (3 × 20 mL), and the combined organic phase was washed with saturated salt water, dried with anhydrous sodium sulfate, concentrated to remove solvent, and finally purified and separated by column chromatography [eluent: V (petroleum ether): V (ethyl acetate)=5:1] to obtain intermediate 18-a, as a light pink oil, with a yield of 95%. ¹H NMR (400 MHz, Chloroform-d) δ 6.37 (m, 2H), 6.31 (s,1H), 3.78 (m, 6H), 3.68 (t, J = 7.8 Hz, 2H), 2.66 (m, 2H), 1.89 (t, J = 7.3 Hz, 2H), 1.27 (s, 1H).

To a solution of intermediate 18-a (1.1 g, 5.61 mmol) in 30 mL of dichloromethane cooled to -78 °C was added dropwise boron tribromide (33.6 mL, 1M in DCM, 33.6 mmol) within 30 minutes, then gradually warmed to room temperature, reacted for 3 hours, and traced and detected with TLC. After the reaction was completed, the reaction solution was cooled to room temperature and added dropwise with water(10 mL), and extracted with ethyl acetate (3 × 10 mL). The combined organic phase was washed with saturated salt water, dried with anhydrous sodium sulfate, concentrated to remove solvent, and purified and separated by column chromatography [eluent: V (dichloromethane): V (methanol)=20:1] to obtain compound18-b, with a yield of 41%. ¹H NMR (400 MHz, Chloroform-d) δ 6.30-6.24 (m, 2H), 6.21 (s, 1H), 5.12-5.06 (m, 2H), 3.41-3.34 (m, 2H), 2.70-2.61 (m, 2H), 2.16-2.05 (m, 2H).

To a solution of intermediate 18-b (2.64 mmol) in 20 mL of acetone was added potassium carbonate (547 mg, 4 mmol), and benzyl bromide (0.72 mL, 6.07 mmol), then refluxed overnight at 50 °C, and traced and detected with TLC. After the reaction was completed, the reaction solution was cooled to room temperature and filtered with diatomite to remove solid. The filter residue was washed with dichloromethane, and the filtrate was concentrated to remove solvent, and finally separated and purified by column chromatography [eluent:V(petroleum ether):V(ethyl acetate) = 20:1] to obtain intermediate 18-c, yield 57%. ¹H NMR (400 MHz, Chloroform-d) δ 7.46-7.29 (m, 10H), 6.52 -6.43 (m, 3H), 5.05-5.00 (m, 4H), 3.42-3.33 (m, 2H), 2.76-2.66 (m, 2H), 2.19-2.08 (m, 2H).

To a solution of 2-n-pentylfuran (0.38 mL, 2.44 mmol) in 5 mL of THF cooled to 0 °C was added dropwise n-butyl lithium (0.78 mL, 2.4 M in hexane, 1.86 mmol), then reacted for 30 minutes. Then a solution of compound 18-c (337mg, 2.44 mmol) in 3 mL of THF was added and reacted for 5 hours, and traced and detected with TLC. After the reaction was completed, the reaction solution was quenched with 10 mL of saturated ammonium chloride solution. The aqueous phase was extracted with ether (3 × 20 mL). The combined organic phase was washed with saturated salt water, dried with anhydrous sodium sulfate, concentrated to remove solvent and finally separated and purified by column chromatography [eluent: V (petroleum ether): V (ethyl acetate)=20:1] to obtain intermediate 18-d. ¹H NMR (400 MHz, Chloroform-d) δ 7.36-7.25 (m, 10H), 6.41-6.36 (m, 3H), 5.82-5.71 (m, 2H), 4.96 -4.94 (m, 4H), 2.61-2.37 (m, 6H), 1.92-1.75 (m, 2H), 1.56-1.51 (m, 2H), 1.28- 1.23 (m, 4H), 0.82 (t, J = 6.8 Hz, 3H).

To a solution of intermediate 18-d (0.144 mmol) in 4 mL of dichloromethane was added N, N-dimethylaniline (0.12 mL, 0.938 mmol) and aluminum trichloride (115.2 mg, 0.864 mmol), then reacted at room temperature for 2 hours, and traced and detected with TLC. After the reaction was completed, the reaction solution was diluted with 5 mL of dichloromethane, then poured into 5 mL of 1N HCL solution and stirred for 5 minutes. The oil phase was separated and the aqueous phase was extracted with dichloromethane (2 × 5 mL). The combined organic phase was washed with 10 mL saturated salt water, dried with anhydrous sodium sulfate, concentrated to remove solvent, and purified by column chromatography to obtain final product 18. ¹H NMR (400 MHz, Chloroform-d) δ 6.28 -6.22 (m, 2H), 6.18 (s, 1H), 5.89-5.82 (m, 2H), 4.86-4.73 (m, 2H), 2.62-2.48 (m, 6H), 1.97-1.84 (m, 2H), 1.71-1.54 (m, 2H), 1.38-1.27 (m, 4H), 0.88 (t, J = 6.8 Hz, 3H).

Compound 19 was synthesized using the same method as in Example 3.

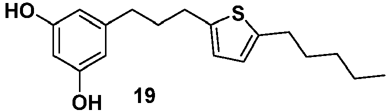

¹H NMR (400 MHz, Chloroform-d) δ 6.58 -6.54 (m, 2H), 6.27-6.21 (m, 2H), 6.17 (s, 1H), 4.92 -4.86 (m, 2H), 2.80-2.71 (m, 4H), 2.55 (t, *J* = 7.6 Hz, 2H), 1.95-1.91 (m, 2H), 1.65-1.61 (m, 2H), 1.36-1.32 (m, 4H), 0.89 (t, *J* = 6.8 Hz, 3H).

### Example 4

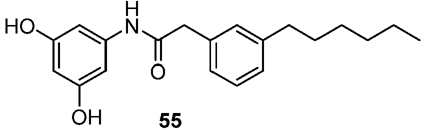

To a flask was added successively 3-iodophenylacetic acid (200 mg, 0.761 mmol), Pd (PPh₃) ₂Cl₂ (16 mg, 0.023 mmol), cuprous iodide (4.4 mg, 0.023 mmol), and triethylamine (0.3 mL),then degassed with N₂ for multiple times, then 1-hexyne (0.1 mL, 0.913 mmol) and acetonitrile (3 mL) was addedand reacted overnight, and traced and detected with TLC. After the reaction was completed, the reaction solution was filtered with diatomite. The diatomite surface was washed with ethyl acetate, and the filtrate was concentrated and finally purified and separated by column chromatography [eluent: V(dichloromethane): V(methanol)=20:1] to obtain intermediate **55-a**, 65 mg, dark oily substance, with a yield of 40%. ¹H NMR (400 MHz, Chloroform-d) δ 10.27 (s, 1H), 7.33-7.15 (m, 4H), 3.59 (s, 2H), 2.39 (t, J = 7.0 Hz, 2H), 1.63-1.41 (m, 4H), 0.94 (t, J = 7.4 Hz, 3H).

To a solution of 5-aminoresorcinol hydrochloride (300 mg, 1.86 mmol) and imidazole (1.01 g, 14.85 mmol) in 7 mL of THF was added a solution of TBSCl (1.12 g) in 7 mL of THF, then reacted at room temperature for 3 hours, and traced and detected with TLC. After the reaction was completed, the reaction solution was concentrated to remove solvent, then added with 15 mL of water and extracted with dichloromethane (3 ×10 mL). The combined organic phase was washed with saturated salt water, dried with anhydrous sodium sulfate, concentrated to remove solvent and finally separated and purified by column chromatography [eluent: V (petroleum ether): V (ethyl acetate)=5:1] to obtain intermediate 55-b, 555mg, colorless oil,with a yield of 85%. 1H NMR (400 MHz, Chloroform-d) δ 5.85-5.83 (m, 2H), 5.78 (t, J = 2.1 Hz, 1H), 3.55 (s, 2H), 0.97-0.95 (m, 18H), 0.19-0.16 (m, 12H).

To a solution of HOBT (41 mg, 0.30 mmol) and EDCI (58 mg, 0.30 mmol) in 1 mL of DCM was added at 0 °C a mixed solution of intermediates 55-a (106 mg, 0.30 mmol) and 55-b (65 mg, 0.30 mmol) in 2 mL of DCM and triethylamine (0.038 mL, 0.3 mmol), then reacted overnight at room temperature, and traced and detected withTLC. After the reaction was completed, the reaction solution was added with 15 mL of water and extracted with EA(3 × 10 mL). The combined organic phase was washed with saturated salt water, dried with anhydrous sodium sulfate, concentrated to remove solvent and finally separated and purified by column chromatography [eluent: V (petroleum ether): V (ethyl acetate)=7:1] to obtain intermediate **55-c**, 88mg, colorless solid, with a yield of 53%. ¹H NMR (400 MHz, Chloroform-d) δ 7.20 -7.15 (m, 2H), 7.12 (t, J = 7.8 Hz, 1H), 7.04 (dt, J = 7.6, 1.7 Hz, 1H), 6.81 (s, 1H), 6.47 -6.42 (m, 2H), 5.90 (s, 1H), 3.46 (s, 2H), 2.23 (t, J = 7.0 Hz, 2H), 1.45-1.36 (m, 2H), 1.35-1.25 (m, 2H), 0.79-0.75 (m, 21H), 0.00- -0.20 (m, 12H).

To a solution of intermediate **55-c** (88mg, 0.159 mmol) in 2 mL of THF was added nBu4N+F- (0.38 mL, 1M in DCM, 0.38 mmol)., then reacted at room temperature for 1 hour, and traced and detected with TLC. After the reaction was completed, the reaction solution was concentrated, then purified and separated by column chromatography [eluent: V(dichloromethane): V(methanol)=15:1] to obtain intermediate 55-d, 40mg, colorless solid, yield 77%. ¹H NMR (400 MHz, Methanol-d4) δ 7.33 (s, 1H), 7.27-7.21 (m, 3H), 6.58 (d, J = 2.2 Hz, 2H), 6.02 (t, J = 2.1 Hz, 1H), 3.59 (s, 2H), 2.40 (t, J = 6.9 Hz, 2H), 1.61-1.43 (m, 4H), 0.96 (t, J = 7.2 Hz, 3H).

To a solution of Intermediate 55-d (0.2 mmol) in a mixed solvent of 2 mL EA and 2 mL MeOH was added palladium carbon, then degassed with N₂ for multiple times, and degassed with H₂ for multiple times, and then reacted overnight at room temperature. After the reaction was completed, the reaction solution was filtered with diatomite, and the filtrate was concentrated, separated by column chromatography[eluent: V (dichloromethane): V (methanol)=20:1] to obtain final product 55. ¹H NMR (400 MHz, Methanol-d4) δ 7.23-7.17 (m, 1H), 7.16-7.09 (m, 2H), 7.08-7.02 (m, 1H), 6.61-6.55 (m, 2H), 6.02 (t, J = 2.2 Hz, 1H), 3.58 (s, 2H), 2.58 (t, J = 7.6 Hz, 2H), 1.6 -1.55 (m, 2H), 1.33-1.27 (m, 6H), 0.87 (t, J = 6.8 Hz, 3H).

### Example 5

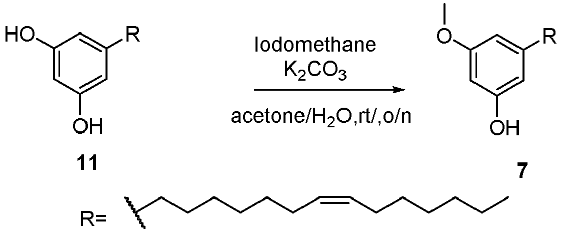

To a solution of compound **11** (38 mg, 0.125 mmol) in 2 mL of acetone was added potassium carbonate (86 mg, 0.624 mmol) and methyl iodide (17.8 mg, 0.125 mmol),then reacted overnight at room temperature, and traced and detected with TLC. After the reaction was completed, the reaction solution was filtered with diatomite, then wash with acetone for multiple times. The filtrate was concentrated to remove solvent and finally purified and separated by column chromatography [eluent: V(petroleum ether): V(ethyl acetate)=10:1] to obtain product **7**, 15 mg, colorless oily substance, with a yield of 38%. ¹H NMR (400 MHz, Chloroform-d) δ 6.32 (s, 1H), 6.28-6.21 (m, 2H), 5.38-5.31 (m, 2H), 4.92 (s, 1H), 3.76 (s, 3H), 2.55-2.47 (m, 2H), 2.04-1.97 (m, 4H), 1.60-1.56 (m, 2H), 1.35-1.24 (m, 14H), 0.88 (t, J = 5.8 Hz, 3H).

### Example 6

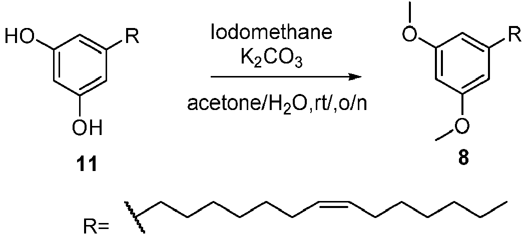

To a solution of compound 11 (38 mg, 0.125 mmol) in 2 mL of acetone was added potassium carbonate (86 mg, 0.624 mmol) and methyl iodide (89 mg, 0.624 mmol), then reacted overnight at room temperature, and traced and detected with TLC. After the reaction was completed, the reaction solution was filtered with diatomite and the diatomite was wash with acetone for multiple times. The filtrate was concentrated to remove solvent and finally purified and separated by column chromatography [eluent: V(petroleum ether): V(ethyl acetate)=30:1] to obtain product **8**, 33 mg, colorless oily substance, with a yield of 80%. ¹H NMR (400 MHz, Chloroform-d) δ 6.37-6.32 (m, 2H), 6.29 (s, 1H), 5.40- 5.28 (m, 2H), 3.80-3.76 (m, 6H), 2.58-2.51 (m, 2H), 2.07-1.96 (m, 4H), 1.65-1.57 (m, 2H), 1.35-1.26 (m, 14H), 0.86 (t, 5.6 Hz, 3H).

### Example 7

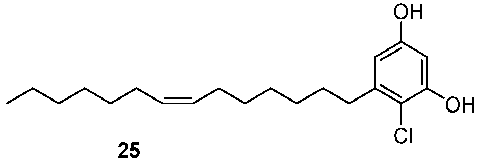

To a solution of phenolic compound (0.164 mmol) and NCS (26 mg, 0.197 mmol) in 0.6 mL of dichloromethane was added DMSO (0.0023 mL, 0.0328 mmol,then reacted at room temperature for 3 hours, and traced and detected with TLC. After the reaction was completed, the reaction solution was concentrated, and separated and purified by column chromatography [eluent: V (petroleum ether): V (ethyl acetate)=4:1] to obtain the corresponding product 25. ¹H NMR (400 MHz, Chloroform-d) δ 6.39 (s, 1H), 6.30 (s, 1H), 5.62 (s, 1H), 5.42-5.28 (m, 2H), 4.75 (s, 1H), 2.68-2.58 (m, 2H), 2.05-1.99 (m, 4H), 1.62-1.55 (m, 2H), 1.41-1.28 (m, 14H), 0.88 (d, J = 5.6 Hz, 3H).

Compound 28 was synthesized using the same method as in Example 7.

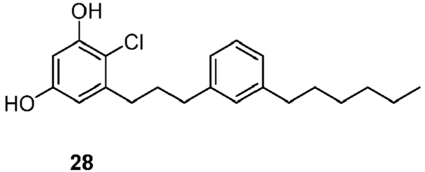

¹H NMR (400 MHz, Chloroform-d) δ 7.18 (t, *J* = 7.7 Hz, 1H), 7.02-6.99 (m, 3H), 6.40 (d, *J* = 2.9 Hz, 1H), 6.30 (d, *J* = 2.9 Hz, 1H), 5.61 (s, 1H), 4.74 (s, 1H), 2.71-2.64 (m, 4H), 2.59-2.55 (m, 2H), 1.94-1.90 (m, 2H), 1.34-1.27 (m, 8H), 0.89 (t, *J* = 6.4 Hz, 3H).

### Example 8

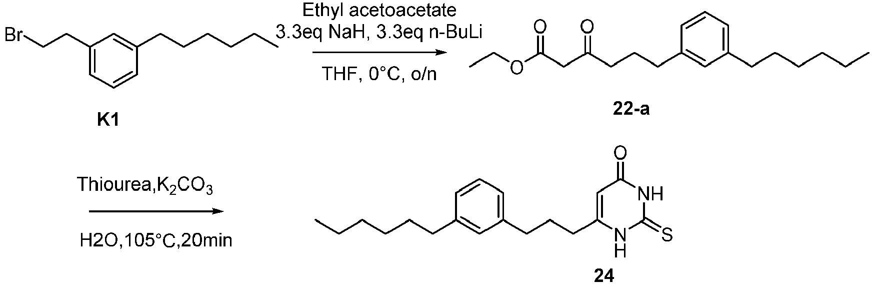

To a solution of ethyl acetoacetate (46 mg, 0.355 mmol) in 2 mL of THF was added NaH (10 mg, 0.391 mmol) at 0 °C, then stirred for 5 minutes, n-butyl lithium (0.17 mL, 2.4 M in hexane, 0.391 mmol) was then added and continued to react for 5 minutes. Then a solution of **K1** (100 mg, 0.355 mmol) in 1 mL of THF was added, and stirred for 12 hours at 0 °C, and traced and detected with TLC. After the reaction was completed, the reaction was quenched with 5 mL of water and extracted with EA(3 × 5 mL). The combined organic phase was washed with saturated salt water, dried with anhydrous sodium sulfate, concentrated to remove solvent and finally separated and purified by column chromatography [eluent: V (petroleum ether): V (ethyl acetate)=20:1] to obtain intermediate **22-a**, 70 mg, colorless oil, with a yield of 60%. ¹H NMR (400 MHz, Chloroform-d) δ 7.18 (dd, J = 8.9, 6.7 Hz, 1H), 7.02 -6.95 (m, 3H), 4.19 (q, J = 7.2 Hz, 2H), 3.41 (s, 2H), 2.62-2.53 (m, 6H), 1.66-1.61 (m, 4H), 1.36-1.24 (m, 6H), 0.88 (t, J = 6.8 Hz, 3H)_{∘}

To a heated (70 °C) solution of thiourea (23 mg, 0.30 mmol) in 0.5 mL of water was addeed potassium carbonate (42 mg, 0.30 mmol) and then transferred to **22-a** (62 mg, 0.193 mmol) and reacted at 105°C. After the solvent was evaporated, the reaction residue was cooled to room temperature and dissolved with 0.5 mL of water. The resulting solution appeared milky white and 1N HCl was added to adjust the pH to acidity with the precipitation of white viscous solid. The supernatant was discarded. The solid was washed with water (2 × 3 mL), dissolved with 2 mL of EA, dried with anhydrous sodium sulfate, and purified and separated by column chromatography [eluent: V (petroleum ether): V (ethyl acetate)=2:1] to obtain product **24**, 14 mg, white solid, with a yield of 24%. ¹H NMR (400 MHz, Chloroform-d) δ 10.61-10.50 (m, 2H), 7.19 (t, J = 7.8 Hz, 1H), 7.05 -6.94 (m, 3H), 5.75 (s, 1H), 2.66-2.53 (m, 4H), 2.43 (t, J = 7.0 Hz, 2H), 1.62-1.55 (m, 2H), 1.37-1.28 (m, 8H), 0.88 (t, J = 6.8 Hz, 3H).

### Example 9

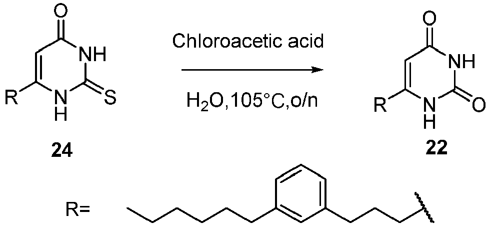

To a solution of chloroacetic acid (53 mg, 0.558 mmol) in 1 mL of water was slowly added dropwise a solution of **25** (92 mg, 0.279 mmol) in 0.5 mL of THF, then reacted overnight at 100 °C, cooled to room temperature, added dropwise with 0.2 mL of concentrated HCl. The reaction was reheated and refluxed, and traced and detected with TLC. After the reaction was completed, the reaction solution was extracted with EA(3 × 2 mL). The combined organic phase was washed with saturated salt water, dried with anhydrous sodium sulfate, concentrated to remove solvent and finally separated and purified by column chromatography [eluent: V (dichloromethane): V (methanol)=20:1] to obtain product **22**, 3.2 mg, white solid, with a yield of 3%. 1H NMR (400 MHz, Methanol-*d₄*) δ 7.17 (t, J = 7.5 Hz, 1H), 7.04-6.97 (m, 3H), 5.44 (s, 1H), 2.65 (t, J = 7.6 Hz, 2H), 2.57 (t, J = 7.6 Hz, 2H), 2.40 (t, J = 7.6 Hz, 2H), 1.96-1.85 (m, 2H), 1.63-1.59 (m, 2H), 1.35-1.29 (m, 6H), 0.89 (t, J = 7.0 Hz, 3H).

### Example 10

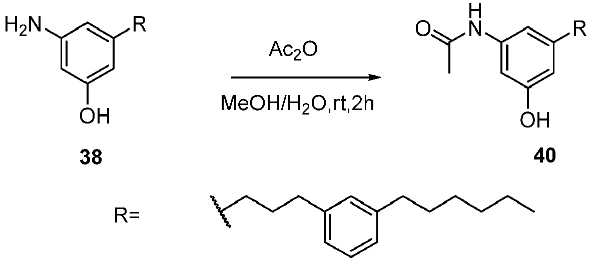

To a suspension of compound **38** (18 mg, 0.0578 mmol) in 0.5 mL of water was added dropwise a solution of acetic anhydride(13 mg, 0.133 mmol) in 1 mL of methanol, then reacted for 2 hours at room temperature, and traced and detected with TLC. After the reaction was completed, the reaction solution was cooled to room temperature, concentrated to remove solvent, and dump the top layer of water, and purified and separated by column chromatography [eluent: V(dichloromethane): V(methanol)=20:1] to obtain product 40, 16 mg, colorless oil, with a yield of 90%. ¹H NMR (400 MHz, Chloroform-d) δ 8.39 (s, 1H), 7.78 (s, 1H), 7.31 (s, 1H), 7.17 (t, J = 7.7 Hz, 1H), 7.04-6.94 (m, 3H), 6.50 (s, 1H), 6.34 (s, 1H), 2.61-2.53 (m, 6H), 2.18 (s, 3H), 1.95-1.89 (m, 2H), 1.62-1.55 (m, 2H), 1.38 -1.27 (m, 6H), 0.88 (t, J = 5.6 Hz, 3H).

### Example 11

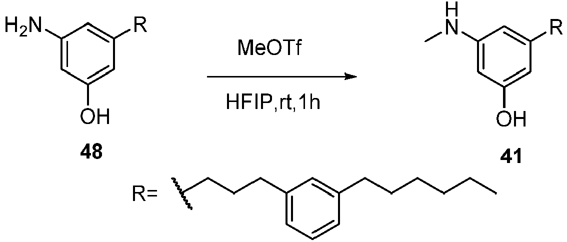

To a solution of compound **38** (26 mg, 0.0836 mmol) in 0.5 mL of HFIP was added MeOTf (0.014 mL, 0.125 mmol), then reacted at room temperature for 1 hour, and traced and detected by TLC. After the reaction was completed, the reaction was quenched with 2 mL of 1 N HCl and extracted with EA(3 × 2 mL). The combined organic phase was washed with saturated salt water, dried with anhydrous sodium sulfate, concentrated to remove solvent and finally separated and purified by column chromatography [eluent: V (petroleum ether): V (ethyl acetate)=4:1] to obtain product **41**, 6.3 mg, brown red oil, with a yield of 24%. ¹H NMR (400 MHz, Chloroform-d) δ 7.18(t, 8.0 Hz, 1H), 7.03-6.97 (m, 3H), 6.08-6.01 (m, 2H), 5.97-5.93 (m, 1H), 2.80 (s, 3H), 2.63-2.50 (m, 6H), 1.97-1.85 (m, 2H), 1.63-1.57 (m, 2H), 1.37-1.29 (m, 6H), 0.90-0.86 (m, 3H).

### Example 12

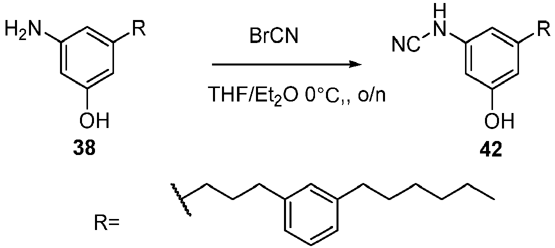

To a flask was added compound 38 (33 mg, 0.106 mmol), 1 mL of THF and 0.5 mL of anhydrous ether, then slowly added to a solution of BrCN in 0.5 mL of THF at 0 °C, and reacted overnight at room temperature, and traced and detected by TLC. After the reaction was completed, the reaction solution was concentrated and separated by column chromatography [eluent: V(dichloromethane): V(methanol)=20:1] to obtain final product **42**, 15 mg, oil, with a yield of 42%. ¹H NMR (400 MHz, Chloroform-d) δ 7.19 (t, J = 7.8 Hz, 1H), 7.03-6.96 (m, 3H), 6.42-6.39 (m, 2H), 6.34 (t, J = 1.7 Hz, 1H), 6.12-5.48 (m, 2H), 2.64-2.51 (m, 6H), 1.97-1.85 (m, 2H), 1.63-1.56 (m, 2H), 1.36-1.29 (m, 6H), 0.88 (t, J = 6.6 Hz, 3H).

### Example 13

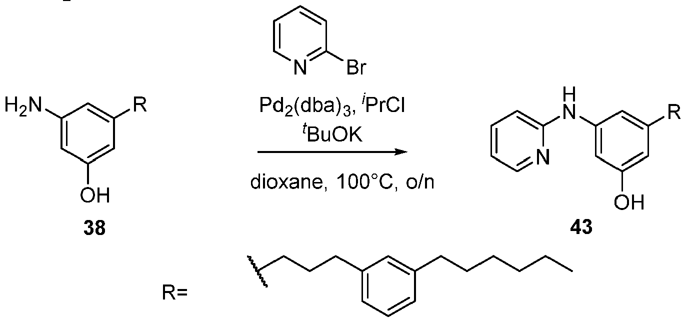

To a three-neck bottle was added compound **38** (33 mg, 0.106 mmol), potassium t-butoxide (36 mg, 0.318 mmol), Pd₂(dba)₃ (10 mg, 0.011 mmol), and iPrCl (10 mg, 0.080 mmol), then degassed with N₂ for three times and 1-bromopyridine (0.012 mL, 0.127 mmol) and dioxane (2 mL) were added, and then reacted overnight at 100 °C, and traced and detected by TLC. After the reaction was completed, the reaction was quenched with 5 mL of water and extracted with EA(3 × 5 mL). The combined organic phase was washed with saturated salt water, dried with anhydrous sodium sulfate, concentrated to remove solvent and finally separated and purified by column chromatography [eluent: V (dichloromethane): V (methanol)=20:1] to obtain product **43**, 20mg, brown solid, with a yield of 45%. ¹H NMR (400 MHz, Chloroform-d) δ 8.16 (dd, J = 5.2, 1.8 Hz, 1H), 7.46 (ddd, J = 8.7, 7.1, 1.8 Hz, 1H), 7.17 (t, J = 8.6Hz, 1H), 7.01-6.96 (m, 3H), 6.89 (dd, J = 8.7,2.2 Hz, 1H), 6.75-6.67 (m, 2H), 6.62-6.55 (m, 2H), 6.40 (t, J = 1.8 Hz, 1H), 2.63-2.51 (m, 6H), 1.95-1.85 (m, 2H), 1.63-1.54 (m, 2H), 1.34-1.24 (m, 8H), 0.88 (t, J = 6.6 Hz, 3H).

### Example 14

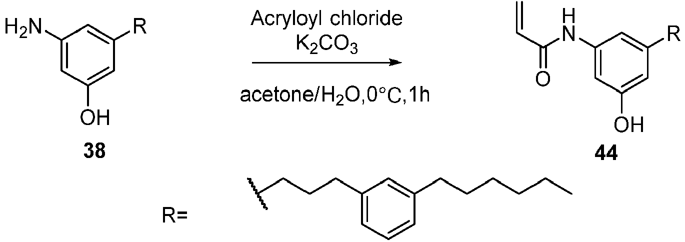

To a solution of 0.8 mL of acetone and 0.2 mL of water was added compound 38 (35 mg, 0.113 mmol) and potassium carbonate (30.4 mg, 0.225 mmol). The mixture was added with acryloyl chloride (10.1 mg, 0.113 mmol) at 0 °C, then stirred at room temperature for 1 hour, and traced and detected by TLC. After the reaction was completed, the reaction was quenched with 3 mL of water and extracted with EA(3 × 3 mL). The combined organic phase was washed with saturated salt water, dried with anhydrous sodium sulfate, concentrated to remove solvent and finally separated and purified by column chromatography [eluent: V (dichloromethane): V (ethyl methanol)=20:1] to obtain product **44**, 20 mg, colorless oil, with a yield of 48%. ¹H NMR (400 MHz, Chloroform-d) δ 8.09 (s, 1H), 7.83 (s, 1H), 7.37 (s, 1H), 7.17 (t, J = 7.7 Hz, 1H), 7.02-6.95 (m, 3H), 6.53 (t, J = 1.8 Hz, 1H), 6.47-6.40 (m, 2H), 6.22 (dd, J = 16.9, 10.4 Hz, 1H), 5.79 (d, J = 10.4 Hz, 1H), 2.62-2.53 (m, 6H), 1.96-1.86 (m, 2H), 1.63-1.53 (m, 2H), 1.34-1.27 (m, 6H), 0.87 (t, J = 5.6 Hz, 3H).

### Example 15

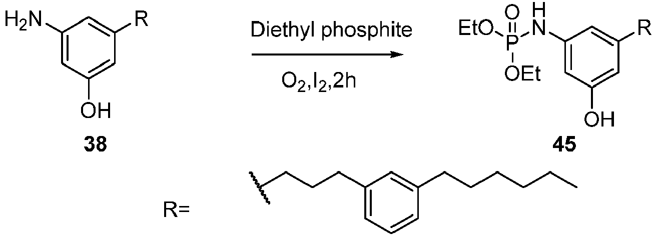

To an open mouthed flask was added Compound **38** (25.8, 0.083 mmol) and iodine (1 mg, 0.0013 mmol), thendiethyl phosphite(23 mg, 0.166 mmol) was added dropwise at room temperature, and traced and detected by TLC. After the reaction was completed, the reaction solution was concentrated, and separated and purified by column chromatography [eluent: V (petroleum ether): V (ethyl acetate)=3:1] to obtain product 45, 4.0 mg, with a yield of 13%. ¹H NMR (400 MHz, Chloroform-d) δ 7.17 (t, J = 7.7 Hz, 1H), 7.02-6.95 (m, 3H), 6.47 (t, J = 2.2 Hz, 1H), 6.34-6.30 (m, 2H), 5.44-5.35 (m, 2H), 4.20-4.04 (m, 4H), 2.63-2.48 (m, 6H), 1.94-1.84 (m, 2H), 1.61-1.56 (m, 2H), 1.33-1.27 (m, 12H), 0.90-0.85 (m, 3H).

### Example 16

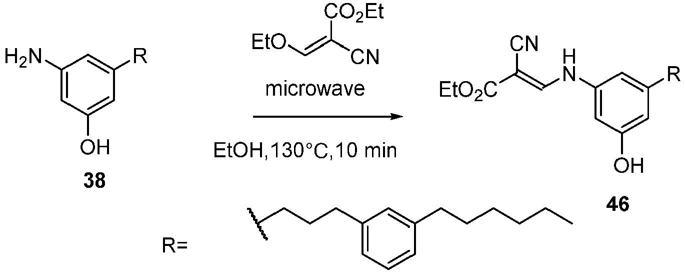

Compound **38** (33 mg, 0.106 mmol) and ethyl (ethoxymethylene)cyanoacetate (19 mg, 0.110 mmol) were dissolved in 0.4 mL of EtOH and the mixture was subjected to microwave reaction for 10 minutes, and traced and detected by TLC. After the reaction was completed, the reaction solution was concentrated, and separated and purified by column chromatography [eluent: V (petroleum ether): V (ethyl acetate)=3:1] to obtain a mixture **46** of cis-trans isomers. ¹H NMR (400 MHz, Chloroform-d) δ 10.66 (d, J = 13.5 Hz, 1H), 7.81 (d, J = 13.5 Hz, 1H), 7.19 (t, J = 7.8 Hz, 1H), 7.04-6.95 (m, 3H), 6.53-6.41 (m, 3H), 5.72 (s, 1H), 4.29 (q, J = 7.1 Hz, 2H), 2.64-2.54 (m, 6H), 1.98-1.87 (m, 2H), 1.64-1.54 (m, 2H), 1.40-1.24 (m, 9H), 0.88 (t, J = 6.6 Hz, 3H).

### Example 17

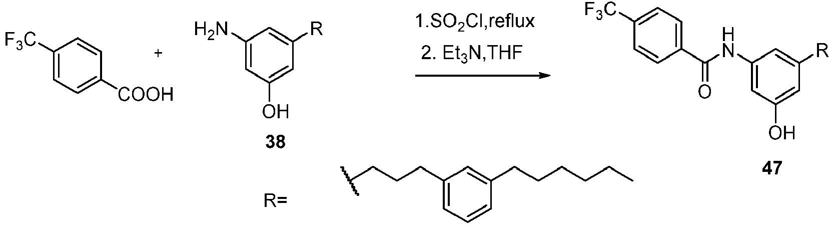

P-trifluoromethylbenzoic acid (1.0 g, 5.26 mmol) was dissolved in 2 mL of thionyl chloride, then heated and refluxed for 2 hours. The reaction solution was concentrated to remove solvent, and obtain acyl chloride compound. Compound 38 (32.5 mg, 0.104 mmol) was dissolved in 1 mL of THF, then degassed with N₂ for multiple times, followed by the addition of triethylamine (0.030 mL, 0.209 mmol) and acyl chloride (22 mg, 0.104 mmol) at 0 °C. The reaction was carried out overnight at room temperature, and traced and detected by TLC. After the reaction was completed, the reaction solution was concentrated, and separated and purified by column chromatography [eluent: V (petroleum ether): V (ethyl acetate)=5:1] to obtain product 47, 25 mg, brown red solid, with a yield of 50%. ¹H NMR (400 MHz, Chloroform-d) δ 7.96-7.85 (m, 3H), 7.78-7.71 (m, 3H), 7.45 (s, 1H), 7.17 (t, J = 7.8 Hz, 1H), 7.03-6.94 (m, 3H), 6.59 (t, J = 1.7 Hz, 1H), 6.55 (t, J = 1.7 Hz, 1H), 2.63-2.53 (m, 6H), 1.97-1.87 (m, 2H), 1.63-1.54 (m, 2H), 1.33-1.25 (m, 6H), 0.87 (t, J = 5.6 Hz, 3H).

### Example 18

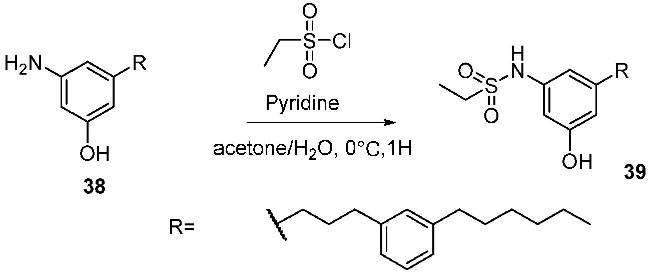

To a solution of compound **38** (18 mg, 0.0554 mmol) in 2 mL of DCM was added pyridine (11.8 mg, 0.150 mmol) and substituted sulfonyl chloride (0.0665 mmol) at 0°C, then reacted overnight at room temperature, and traced and detected by TLC. After the reaction was completed, the reaction solution was concentrated and purified and separated by column chromatography [eluent: V(dichloromethane): V(methanol)=20:1] to obtain compound **39.** ¹H NMR (400 MHz, Chloroform-d) δ 7.61 (t, J = 7.4 Hz, 1H), 7.52 (s, 1H), 7.45-7.38 (m, 3H), 7.12 (t, J = 1.8 Hz, 1H), 7.00 (t, J = 1.8 Hz, 1H), 6.94 (t, J = 1.8 Hz, 1H), 6.75 (s, 1H), 3.57 (q, J = 7.4 Hz, 2H), 3.08-2.93 (m, 6H), 2.39-2.27 (m, 2H), 2.09-1.98 (m, 2H), 1.75 (q, J = 6.5, 5.7 Hz, 9H), 1.31 (t, J = 5.6 Hz, 3H).

The following compounds were synthesized using the same method as in Example 18.

| Compo und No. | Structure | NMR |
|---|---|---|
| **48** | 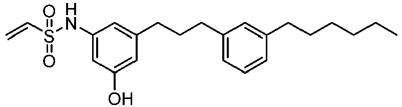 | 1H NMR (400 MHz, Chloroform-d) δ 7.18 (t, J = 7.9 Hz, 1H), 7.03- 6.95 (m, 3H), 6.71-6.52 (m, 3H), 6.52-6.45 (m, 2H), 6.29 (d, J = 16.5 Hz, 1H), 5.95 (d, J = 9.9 Hz, 1H), 2.63-2.49 (m, 6H), 1.96 -1.84 (m, 2H), 1.63 -1.54 (m, 2H), 1.39-1.28 (m, 6H), 0.88 (t, J = 5.6 Hz, 3H). |
| **49** | 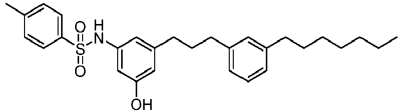 | 1H NMR (400 MHz, Chloroform-d) δ 7.69 -7.63 (m, 2H), 7.15-7.02 (m, 7H), 6.56 (t, J = 2.1 Hz, 1H), 6.44-6.37 (m, 2H), 2.54-2.46 (m, 6H), 2.25 (s, 3H), 1.78-1.69 (m, 2H), 1.56-1.46 (m, 2H), 1.33-1.27 (m, 8H), 0.87 (t, J = 7.0 Hz, 3H). |
| **50** | 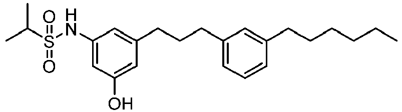 | 1H NMR (400 MHz, Chloroform-d) δ 7.19 (t, J = 7.8 Hz, 1H), 7.03-6.95 (m, 3H), 6.63 (t, J = 2.2 Hz, 1H), 6.53 (t, J = 1.6 Hz, 1H), 6.47-6.44 (m, 1H), 6.22 (s, 1H), 4.92 (s, 1H), 3.37-3.29 (m, 1H), 2.64-2.53 (m, 6H), 1.96-1.86 (m, 2H), 1.64-1.58 (m, 2H), 1.44-1.29 (m, 12H), 0.90-0.85 (m, 3H). |
| **51** | 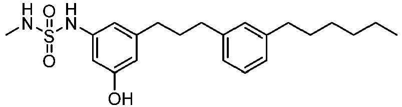 | 1H NMR (400 MHz, Chloroform-d) δ 7.19 (t, J = 7.7 Hz, 1H), 7.07-6.96 (m, 3H), 6.58 (s, 1H), 6.49 (s, 1H), 6.45 (s, 1H), 6.34 (s, 1H), 4.84 (s, 1H), 4.48-4.41 (m, 1H), 2.72 (d, J = 5.4 Hz, 3H), 2.65-2.52 (m, 6H), 1.95-1.88 (m, 2H), 1.65-1.57 (m, 2H), 1.34-1.27 (m, 6H), 0.90-0.86 (m, 3H). |
| **52** | 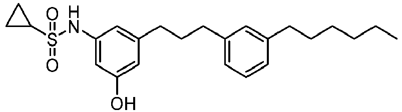 | 1H NMR (400 MHz, Chloroform-d) δ 7.18 (t, J = 7.8 Hz, 1H), 7.04-6.95 (m, 3H), 6.67 (t, J = 2.2 Hz, 1H), 6.61-6.57 (m, 2H), 6.50 (t, J = 1.8 Hz, 1H), 5.46 (s, 1H), 2.63-2.49 (m, 7H), 1.95-1.86 (m, 2H), 1.64-1.55 (m, 2H), 1.36-1.28 (m, 6H), 1.21-1.15 (m, 2H), 1.00-0.92 (m, 2H), 0.90-0.85 (m, 3H). |
| **53** | 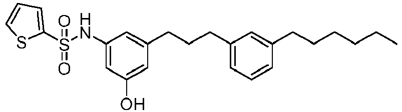 | 1H NMR (400 MHz, Chloroform-d) δ 7.53 -7.46 (m, 2H), 7.18 (t, J = 7.5 Hz, 1H), 7.04-6.91 (m, 4H), 6.70 (s, 1H), 6.57 (t, J = 2.2 Hz, 1H), 6.48-6.42 (m, 2H), 5.13 (s, 1H), 2.59-2.48 (m, 6H), 1.89-1.79 (m, 2H), 1.63- 1.54 (m, 2H), 1.37-1.27 (m, 6H), 0.92-0.84 (m, 3H). |
| **54** | 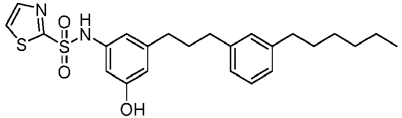 | 1H NMR (400 MHz, Chloroform-d) δ 7.95 (d, J = 3.3 Hz, 1H), 7.80 (s, 1H), 7.50 (d, J = 3.3 Hz, 1H), 7.18 (t, J = 7.4 Hz, 1H), 7.06 -6.90 (m, 3H), 6.64-6.54 (m, 2H), 6.47 (s, 1H), 5.33 (s, 1H), 2.61-2.44 (m, 6H), 1.88 -1.78 (m, 2H), 1.65-1.51 (m, 2H), 1.35-1.26 (m, 6H), 0.88 (d, J = 6.8 Hz, 3H). |

### Example 19 Evaluation of DRAK2 inhibitory activity

### 1. Experiment purpose

To determine the inhibitory activity of compounds against kinases by the kinase assay kit ADP-GLO.

### 2. Experiment principle

DRAK2 is a serine/threonine protein kinase. ADP Glo&# 8482; Kinase Assay (ADP Glo&# 8482; Kinase Assay Kit) is a luminescent kinase assay kit that can detect ADP formed during kinase reactions; this kit can firstly convert ADP into ATP, which is then captured by Ultra Glo&# 8482, luciferase and ultimately converts into light. The emitted light signal is positively correlated with the corresponding ATP, thus through this method, the inhibitory activity of small molecule compounds on DRAK2 can be measured.

### 3. Experimental Sample

The compound was dissolved in DMSO before experiment to prepare stock solution. When using, the stock solution was diluted with culture medium to a desired concentration.

### 4. Experimental methods

I. Kinase Assay
   1. 1 µl of compound was added to the well for each assay
   2. 2 µl of kinase solution was added to each well of the assay plate, except for the control well that does not contain enzymes (changed to add 2 µL of 1x kinase buffer).
   3. 2 µl of ATP was added to each well of the assay plate
   4. the plate was shook and centrifuged.
II. Stop measurement
   2.5 µl ADP-Glo^{™} reagent was add to terminate kinase reaction and consume unconsumed ATP, leaving only ADP and very low ATP background to incubate at room temperature for 60 minutes.
III. Assay analysis
   5 µl of kinase assay reagent was add to convert ADP to ATP, and luciferase and fluorescein were introduced to detect ATP.
IV. Data processing
   Envision was used to measure luminescence
V. Curve fitting
   1. Values were copyied from Envision program
   2. Inhibition percentage = (maximum sampling rate)/(maximum-minimum) X 100.
      The "minimum" refers to the proportion of without enzyme control, while "maximum" refers to the proportion of DMSO control.
   3. GraphPad Prism 5.0 was used to process data
      Through this method, the inhibitory activity of small molecule compounds on DRAK2 can be obtained.

### 5. Experimental results: (Taking four compounds such as compound 1 as examples, but not limited to these compounds)

**Table 1: Test results of DRAK2 inhibitory activity of some compounds**

| Compound No. | DRAK2 (IC₅₀, µM) |
|---|---|
| **1** | 7.73 ± 0.70 |
| **17** | 4.94 ± 1.18 |
| **28** | 4.06 ± 0.35 |
| **39** | 4.93 ± 1.09 |

| | |
|---|---|
| Note: IC₅₀ is the evaluation of inhibitory activity of the sample drugs on DRAK2, and represents half 50% effective concentration. | |

### 6. Summary and discussion

The test results show that these compounds can effectively inhibit the activity of DRAK2 at micromolar level. As a new class of DRAK2 inhibitors, these compounds can be used to develop new therapeutic drugs for metabolic syndrome, including but not limited to diabetes.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A compound of formula I, or a pharmaceutically acceptable salt thereof: 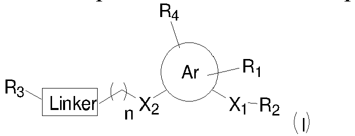 wherein,
R₁ is selected from the group consisting of hydrogen, hydroxyl, halogen, substituted or unsubstituted amino, substituted or unsubstituted C1-C6 alkoxy;
R₂ is selected from the group consisting of hydrogen, CN, C1-C8 alkyl, 5-7 membered heteroaryl, 3-7 membered cycloalkane, C2-C10 alkenyl, - P(O)(ORa)₂, - SO₂Rb, and -(C=O)Rc, preferably hydrogen, cyano, methyl, 5-7 membered heteroaryl, - P(O)(ORa)₂, -SO₂Rb, and -(C=O)Rc, more preferably cyano, pyridine ring, substituted benzene ring, - P(O)(ORa)₂, - SO₂Rb, and -(C=O)Rc;
Ra is independently selected from the group consisting of C1-C8 alkyl, C3-C10 cycloalkyl, 5-7-membered heteroaryl, and C2-C10 alkenyl, preferably C1-C8 alkyl;
Rb is independently selected from the group consisting of C1-C8 alkyl, C3-C10 cycloalkyl, 5-7-membered heteroaryl, C2-C10 alkenyl, preferably C1-C8 alkyl, C3-C10 cycloalkyl, and 5-7-membered heteroaryl, more preferably vinyl, C1-C3 alkyl, C3-C6 cycloalkyl, and 5-membered heteroaryl;
Rc is independently selected from the group consisting of C1-C8 alkyl, C3-C10 cycloalkyl, 5-7-membered heteroaryl, and C2-C10 alkenyl, preferably methyl and vinyl;
R₃ is selected from the group consisting of H, halogen, C1-C10 alkyl, and C3-C6 cycloalkyl;
R₄ is selected from the group consisting of H, OH, and substituted or unsubstituted C1-C8 alkoxy, preferably OH or methoxy;
Ar is selected from the group consisting of substituted or unsubstituted benzene ring, substituted or unsubstituted 5-9 membered heteroaryl, 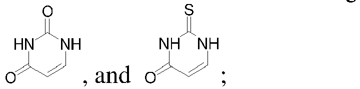
X₁ is a chemical bond, O, NH, NHC(O), NHS(O)₂, NHC(O)NH, NHS(O)₂NH or S;
X₂ is selected from the group consisting of chemical bond, C1-C6 alkylamino carbonyl, and C1-C4 alkyl carbonyl;
n is 0-10, preferably 0-6;
Linker is selected from the group consisting of substituted or unsubstituted C1-C6 alkane, substituted or unsubstituted C1-C6 olefin, substituted or unsubstituted benzene ring, substituted or unsubstituted 5-7-membered heteroaromatic ring, substituted or unsubstituted 3-6 membered cycloalkane, preferably C1-C6 olefin, substituted benzene ring, thiophene ring, and furan ring, more preferably olefin and benzene ring;
unless otherwise specified, the term "substituted" refers to the hydrogen atoms on the group is substituted by one or more substituents selected from the group consisting of halogen, hydroxyl, carboxyl, C1-C6 alkyl, and C2-C10 ester group.

2. The compound according to claim 1, or a pharmaceutically acceptable salt thereof,
wherein, Ar is selected from the group consisting of substituted or unsubstituted benzene ring, substituted or unsubstituted pyridine ring, substituted or unsubstituted indole ring, substituted or unsubstituted pyrimidine ring, substituted or unsubstituted benzimidazole, substituted or unsubstituted indazole, and substituted or unsubstituted benzotriazole, preferably benzene ring or indole ring.

3. The compound according to claim 1, or a pharmaceutically acceptable salt thereof,
wherein, R₂ is selected from the group consisting of hydrogen, cyano, methyl, 5-7-membered heteroaryl, -P(O)(ORa)₂, -SO₂Rb, and -(C=O)Rc;
Ra is independently selected from the group consisting of C1-C8 alkyl;
R_{b} is independently selected from the group consisting of vinyl, C1-C3 alkyl, C3-C6 cycloalkyl, and 5-membered heteroaryl;
Rc is independently selected from the group consisting of methyl and vinyl.

4. The compound according to claim 1, or a pharmaceutically acceptable salt thereof, wherein, R₃ is selected from the group consisting of hydrogen, halogen, isopropyl, and cyclohexane.

5. The compound according to claim 1, or a pharmaceutically acceptable salt thereof, wherein, X₂ is selected from the group consisting of a chemical bond, NHS(O)₂, and -C(=O)-NH-.

6. The compound according to claim 1, or a pharmaceutically acceptable salt thereof, wherein, the compound of formula I has the structure shown in formula II as follows:

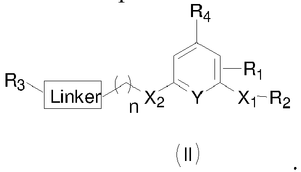

7. The compound according to claim 1, or a pharmaceutically acceptable salt thereof, wherein, the compound is selected from the group consisting of:

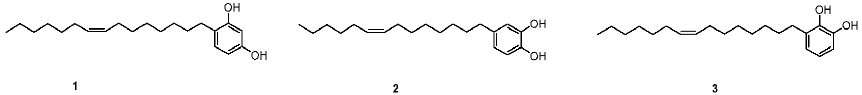

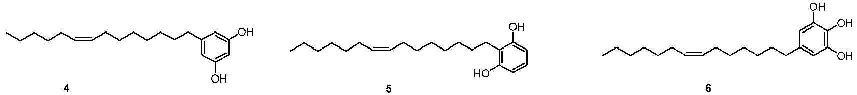

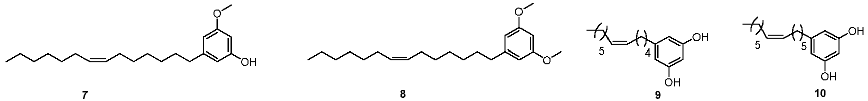

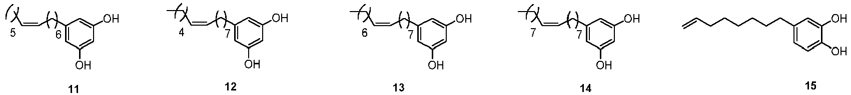

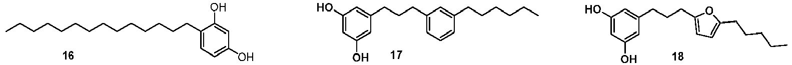

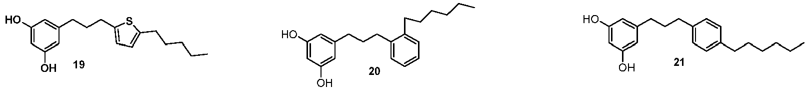

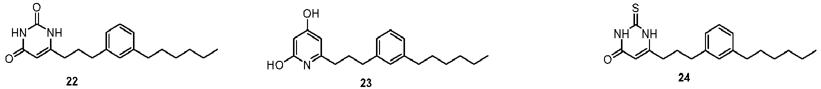

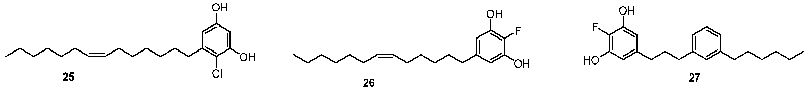

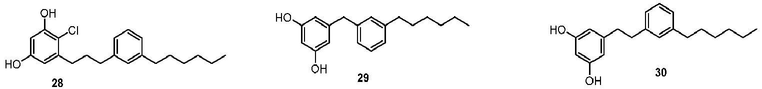

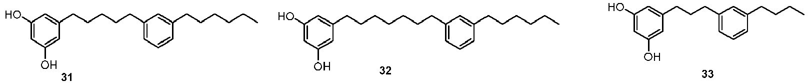

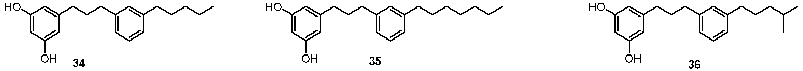

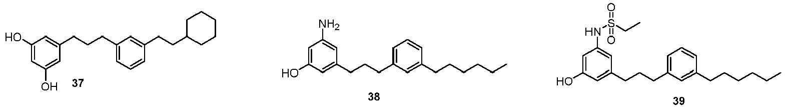

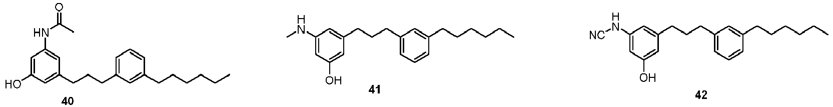

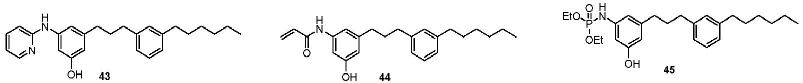

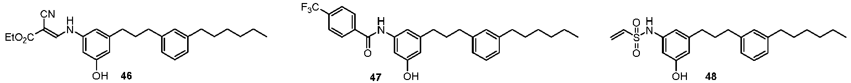

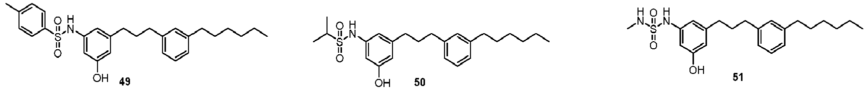

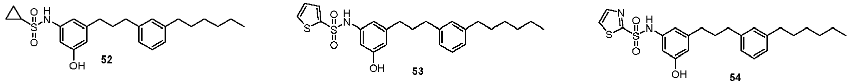

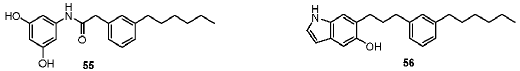

8. A use of the compound according to any one of claims 1-7 in the preparation of a pharmaceutical composition for the treatment or prevention of indications associated with the activity or expression of death associated related apoptotic protein kinase 2 (DRAK2).

9. The use according to claim 8, wherein, the indication is metabolic syndrome.

10. A pharmaceutical composition, wherein the pharmaceutical composition comprises: (1) the compound according to any one of claims 1-7, or pharmaceutically acceptable salts thereof as an active ingredient; and (2) pharmaceutically acceptable carriers.
